# EUROPEAN PATENT SPECIFICATION

(11) **EP 0 964 864 B1**
(45) Date of publication and mention of the grant of the patent: **09.04.2008**
(21) Application number: 98908442.1
(22) Date of filing: 26.01.1998
(51) Int. Cl.: C07D 471/00

(54) **PYRIDO 2,3-D PYRIMIDINES AND 4-AMINOPYRIMIDINES AS INHIBITORS OF CELLULAR PROLIFERATION**
PYRIDO (2,3-D) PYRIMIDINE UND 4-AMINO-PRIMIDINE ALS INHIBITOREN DER ZELLULÄREN PROLIFERATION
PYRIDO 2,3D PYRIMIDINES ET 4-AMINOPYRIMIDINES EN TANT QU'INHIBITEURS DE LA PROLIFERATION CELLULAIRE

(30) Priority: 05.02.1997 US 37220 P; 16.12.1997 US 69743 P
(43) Date of publication of application: 22.12.1999
(60) Divisional application: 07105377.1
(73) Proprietor: Warner-Lambert Company LLC, New York, NY 10017 (US)
(72) Inventor: BOSCHELLI, Diane, Harris, New City, NY 10956 (US); DOBRUSIN, Ellen, Myra, Ann Arbor, MI 48104 (US); DOHERTY, Annette, Marian, F-75016 Paris (FR); FATTAEY, Ali, San Francisco, CA 94115 (US); FRY, David, W., Ypsilanti, MI 48197 (US); BARVIAN, Mark, R., Ann Arbor, MI 48103 (US); TRUMPP-KALLMEYER, Susanne, A, S-43151 Moelndal (SE); WU, Zhipei, Saline, MI 48176 (US)
(74) Representative: Dekker, Henrike Cornelie Christine
(86) International application number: PCT/US1998/001343
(87) International publication number: WO 1998/033798

(56) References cited:
- EP-A- 0 021 292
- EP-A- 0 278 686
- WO-A-92/20642
- WO-A-95/09845
- WO-A-95/15952
- WO-A-96/15128
- WO-A-96/34867
- WO-A-97/38992
- BORREL,J.I. ET AL.: "An unequivocal Synthesis of 4-amino-1,5,6,8-tetra- hydropyrido[2,3-d]pyrimidine-2,7-diones and ..... " COLL.CZECH.CHEM.COMMUN., vol. 61, no. 6, 1996, PRAGUE, pages 901-909, XP002069589
- HURLBERT,B.S. ET AL.: "Studies on Condensed Pyrimidine Systems. XXIII. Synthesis of 2,4-Diaminopyrido[2,3-d]pyrimdiines ..." J.MED.CHEM., vol. 11, 1968, WASHINGTON, pages 703-707, XP002069108
- BAKER,B.R. ET AL.: "Analogs of Tetrahydrofolic acid. XIX. On the Mode of Binding of the Pyrimidyl Moiety ..." J.HETEROCYCLIC.CHEM., vol. 1, 1964, ALBUQUERQUE, pages 263-270, XP002069109
- ANDERSON ET AL.: "Pyridopyrimidines. 6. Nucleophilic Substitutions in the Pyrido[2,3-d]pyrimidine Series" J.ORG.CHEM., vol. 42, 1977, WASHINGTON, page 993 XP002069110
- SCHOFFSTALL,A.M. ET AL.: "Synthesis of 5,6-Dihydropyrido[2,3-d]pyrimidine Derivatives Directly from Acyclic Precursors " J.ORG.CHEM., vol. 36, no. 16, 1971, WASHINGTON, pages 2385-2387, XP002069111
- VICTORY,P. ET AL.: "New Synthesis of pyrido[2,3-d]pyrimidines. 1. Reaction of 6-alkoxy-5-cyano-3,4-dihydro-2-pyridones with Guanidine and Cyanamide " HETEROCYCLES, vol. 23, no. 5, 1985, pages 1135-1141, XP002069590
- VICTORY,P. ET AL.: "Cyclisation of Dinitriles by hydrogen halides. 1. Hydrogen bromide." HETEROCYCLES, vol. 23, no. 8, 1985, pages 1947-1950, XP002069591
- VICTORY,P. ET AL.: "Nueva sintesis de pirido[2,3-d]pirimidinas. V. Deshidrogenacion del anillo dihidropiridonico ...." AFINIDAD, vol. 46, March 1989, pages 107-113, XP002069623
- REWCASTLE G W ET AL: "TYROSINE KINASE INHIBITORS. 10. ISOMERIC 4- (3-BROMOPHENYL)AMINOPYRIDO D-PYRIMIDINES ARE POTENT ATP BINDING SITE INHIBITORS OF THE TYROSINE KINASE FUNCTION OF THE EPIDERMAL GROWTH FACTOR RECEPTOR" JOURNAL OF MEDICINAL CHEMISTRY, vol. 39, no. 9, 1996, pages 1823-1835, XP002046257

## Description

### FIELD OF THE INVENTION

This invention relates to pyridopyrimidines and 4-aminopyrimidines that inhibit cyclin-dependent kinase and growth factor-mediated kinase enzymes, and as such are useful to treat cell proliferative disorders such as atherosclerosis, restenosis, and cancer.

### SUMMARY OF THE RELATED ART

Cell cycle kinases are naturally occurring enzymes involved in regulation of the cell cycle (Meijer L., "Chemical Inhibitors of Cyclin-Dependent Kinases", Progress in Cell Cycle Research, 1995;1:351-363). Typical enzymes include the cyclin-dependent kinases (cdk) cdk1, cdk2, cdk4, cdk5, cdk6, and wee-1 kinase. Increased activity or temporally abnormal activation of these kinases has been shown to result in development of human tumors and other proliferative disorders such as restenosis. Compounds that inhibit cdks, either by blocking the interaction between a cyclin and its kinase partner, or by binding to and inactivating the kinase, cause inhibition of cell proliferation, and are thus useful for treating tumors or other abnormally proliferating cells.

Several compounds that inhibit cdks have demonstrated both preclinical and clinical anti-tumor activity. For example, flavopiridol is a flavonoid that has been shown to be a potent inhibitor of several types of breast and lung cancer cells (Kaur, et al., J. Natl. Cancer Inst., 1992;84:1736-1740; Int. J. Oncol., 1996;9:1143-1168). The compound has been shown to inhibit cdk2 and cdk4. Olomoucine [2-(hydroxyethylamine)-6-benzylamine-9-methylpurine] is a potent inhibitor of cdk2 and cdk5 (Vesely, et al., Eur. J. Biochem., 1994;224:771-786), and has been shown to inhibit proliferation of approximately 60 different human tumor cell lines used by the National Cancer Institute (NCI) to screen for new cancer therapies (Abraham, et al., Biology of the Cell, 1995;83:105-120).

Despite the progress that has been made, the search continues for small molecular weight compounds that are orally bioavailable and useful for treating a wide variety of human tumors and other proliferative disorders such as restenosis and atherosclerosis.

### SUMMARY OF THE INVENTION

This invention provides pyridopyrimidines and 4-aminopyrimidines that are useful for treating cell proliferative disorders, such as cancer, atherosclerosis, restenosis, psoriasis, and endometriosis. We have discovered a group of 7,8-dihydro-2-(amino and thio)-7-(oxo, thio, or imino)-pyrido[2,3-d]pyrimidines and 4-aminopyrimidines that are potent inhibitors of cyclin-dependent kinases (cdks). The compounds are readily synthesized and can be administered by a variety of routes, including orally and parenterally, and have little or no toxicity. The compounds of the invention are members of the class of compounds of 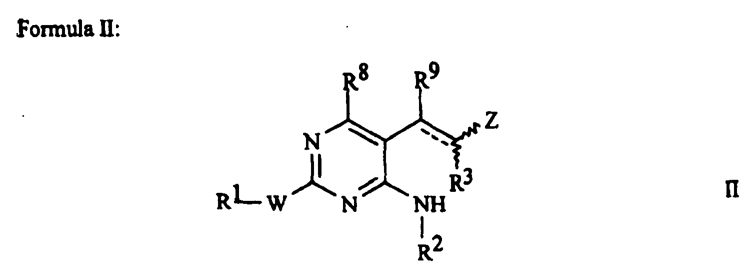 wherein:
W is NH, S, SO, or SO₂;
R¹ and R² include alkyl, cycloalkyl, substituted alkyl, and substituted cycloalkyl;
R³ includes hydrogen, alkyl, and halogen;
X is O, S, or NH;
R⁸ and R⁹ independently are hydrogen, alkyl, alkoxy, halo, amino, and the like; and pharmaceutically acceptable salts thereof.

This invention also provides pharmaceutical formulations comprising a compound of Formula II together with a pharmaceutically acceptable carrier, diluent, or excipient therefor.

Compounds within the scope of the present invention are inhibitors of the cyclin-dependent kinases such as cdk2, cdc2, and cdk4. Some of the compounds of the present invention also inhibit growth factor mediated tyrosine kinases including platelet-derived growth factor (PDGF), fibroblast growth factor (FGF), and epidermal growth factor (EGF). As inhibitors of cyclin-dependent, as well as growth factor-mediated, tyrosine kinases, the compounds of the instant invention are useful in controlling proliferative disorders such as cancer, psoriasis, vascular smooth muscle cell proliferation associated with atherosclerosis, and postsurgical vascular stenosis and restenosis in mammals.

A further embodiment of this invention is a method of treating subjects suffering from diseases caused by cellular proliferation. The method entails inhibiting proliferation of tumorigenic cells of epithelial origin and vascular smooth muscle proliferation, and/or cellular migration by administering a therapeutically effective amount of a compound of Formula II to a subject in need of treatment.

A further embodiment of this invention is a method of treating subjects suffering from diseases caused by DNA tumor viruses such as herpes viruses.

### DETAILED DESCRIPTION OF THE INVENTION

We have discovered a new class of compounds that are potent inhibitors of cyclin-dependent kinases (cdks) and are useful agents for treating subjects suffering from diseases caused by abnormal cell proliferation. Compounds within the scope of the present invention the inhibitors of the cyclin-dependent kinases such as cdc2, cdk2, and cdk4. As inhibitors of cyclin-dependent kinases, the compounds of the instant invention are useful in controlling proliferative disorders such as cancer, psoriasis, vascular smooth muscle proliferation associated with atherosclerosis, postsurgical vascular stenosis, and restenosis in mammals.

The compounds of the invention comprise those of Formula II: 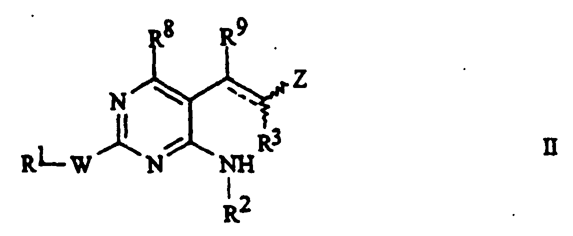 wherein:
the dotted line represents an optional double bond of either trans or cis-stereochemistry;
W is NH, S, SO, or SO2;
Z is COOR⁷, CN, CHO, CH₂OR⁷, CH₂NHR⁷, CONHR⁷, or COR⁷;
R 1 and R 2 are independently selected from the group consisting of H,
(CH₂)ₙAr wherein aryl Is selected from phenyl or naphthyl (CH₂)ₙhoteroaryl, wherein the heteroaryl group have from 4 to 9 ring atoms, from 1 to 4 of which are independently selected from the group consisting of O, S, and N, (CH₂)ₙheterocycle, which means a cycloalkyl group as defined below having at least one hetero atom selected from 0, S, NR₂,
C₁-C₁₀ alkyl,
C₃-C₁₀ cycloalkyl,
C₂₋C₁₀ alkenyl, and
C₂-C₁₀ alkynyl,
wherein n is 0, 1, 2, or 3 and the
(CH₂)ₙAr, alkyl, cycloalkyl, alkenyl, and alkynyl groups are optionally substituted by groups of
NR⁴R⁵,
N(O)R⁴R⁵,
NR⁴R⁵R⁶Y,
phenyl,
phenyl substituted by 1, 2, or 3 groups independently selected from the group consisting of C₁-C₁₀ alkyl, C₁-C₁₀ alkoxy, thio, thio C₁-C₁₀ alkyl, hydroxy, -COOR⁷, amino of the formula -NR⁴R⁵, and T(CH₂)ₘQR⁴ or T(CH₂)ₘCO₂R⁴ wherein m is 1 to 8, T is O, S, NR⁴, N(O)R⁴, NR₄R⁶Y, or CR⁴R⁵, Q is O, S, NR⁵, N(O)R⁵, or NR⁵R⁶Y, hydroxy,
C₁-C₁₀ alkoxy,
Phenoxy,
thiol,
thioC₁-C₁₀alkyl
halo,
COR⁴,
CO₂R⁴,
CONR⁴R⁵,
SO₂NR⁴R⁵,
SO₃R⁴,
PO₃R⁴,
aldehyde,
nitrile,
nitro,
heteroaryloxy wherein heteroaryl is as defined above,
T(CH₂)ₘQR⁴, C(O)T(CH₂)ₘQR⁴, NHC(O)T(CH₂)ₘQR⁴, or T(CH₂)ₘCO₂R⁴ wherein m is 1 to 8, T is O, S, NR⁴, N(O)R⁴, NR⁴R⁵Y, or CR⁴R⁵, Q is O, S, NR⁵, N(O)R^{5,} orNR⁵R⁶Y
R³ is H or C₁-C₁₀alkyl
R⁴ and R⁵ are independently selected from the group consisting of
hydrogen,
C₁-C₆ alkyl
substituted alkyl as defined above
C₂-C₆ alkenyl,
C₂-C₆ alkynyl,
(CH₂)ᵣAr as defined above
C₃-C₁₀ cycloalkyl,
or
R⁴ and R⁵ taken together with the nitrogen to which they are attached optionally form a ring having 3 to 7 carbon atoms and said ring optionally contains 1, 2, or 3 heteroatoms selected from the group consisting of
nitrogen,
substituted nitrogen wherein "substituted nitrogen" means nitrogen bearing C₁-C₆ alkyl or (CH₂)ₙPh where n is 1, 2, or 3,
oxygen, and
sulfur;
R⁶ is C₁-C₁₀ alkyl;
Y is a halo counter-ion;
R⁷ is one of H, C₁-C₁₀ alkyl, or phenyl;
R⁸ and R⁹ independently are
H,
C₁-C₃alkyl,
NR⁴R⁵,
N(O)R⁴R⁵,
NR⁴R⁵R⁶Y,
hydroxy,
alkoxy as defined above ,
thiol,
thioalkyl as defined above ,
halo,
COR⁴, CO²R⁴,
CONR⁴R⁵, SO₂NR⁴R⁵, SO₃R⁴, PO₃R⁴, CHO, CN, or NO₂;
and the pharmaceutically acceptable salts thereof.

Preferably, compounds of Formula II have a trans double bond between C₅ and C₆, more preferably with R¹ being phenyl, and even more preferably with both R¹ being phenyl and R² being alkyl or cycloalkyl.

Also preferred are compounds of Formula II wherein R⁸ and R⁹ both are hydrogen.

Examples of NR⁴R⁵ groups include amino, methylamino, di-isopropylamino, acetyl amino, propionyl amino, 3-aminopropyl amino, 3-ethylaminobutyl amino, 3-di-n-propylamino-propyl amino, 4-diethylaminobutyl amino, and 3-carboxypropionyl amino. R⁴ and R⁵ can be taken together with the nitrogen to which they are attached to form a ring having 3 to 7 carbon atoms and 1,2, or 3 heteroatoms selected from the group consisting of nitrogen, substituted nitrogen, oxygen, and sulfur. Examples of such cyclic NR⁴R⁵ groups include pyrrolidinyl, piperazinyl, 4-methylpiperazinyl, 4-benzylpiperazinyl, pyridinyl, piperidinyl, pyrazinal, morpholinyl, and the like.

Unless otherwise expressly stated, the following definitions are adhered to throughout this disclosure.

"Alkyl" means a straight or branched hydrocarbon radical having from 1 to 10 carbon atoms (unless stated otherwise) and includes, for example, methyl, ethyl, n-propyl, isopropyl, n-butyl, sec-butyl, isobutyl, tert-butyl, n-pentyl, isopentyl, n-hexyl, and the like.

"Halo" includes fluoro, chloro, bromo, and iodo.

"Alkenyl" means straight and branched hydrocarbon radicals having from 2 to 6 carbon atoms and one double bond and includes ethenyl, 3-buten-1-yl, 2-ethenylbutyl, 3-hexen-1-yl, and the like.

"Alkynyl" means straight and branched hydrocarbon radicals having from 2 to 6 carbon atoms and one triple bond and includes ethynyl, 3-butyn-1-yl, propynyl, 2-butyn-1-yl, 3-pentyn-1-yl, and the like.

"Cycloalkyl" means a monocyclic or polycyclic hydrocarbyl group such as cyclopropyl, cycloheptyl, cyclooctyl, cyclodecyl, cyclobutyl, adamantyl, norpinanyl, decalinyl, norbornyl, cyclohexyl, and cyclopentyl. Such groups can be substituted with groups such as hydroxy, keto, and the like. Also included are rings in which 1 to 3 heteroatoms replace carbons. Such groups are termed "heterocyclyl", which means a cycloalkyl group also bearing at least one heteroatom selected from O, S, or NR₂, examples being oxiranyl, pyrrolidinyl, piperidyl, tetrahydropyran, and morpholine.

"Alkoxy" refers to the alkyl groups mentioned above bound through oxygen, examples of which include methoxy, ethoxy, isopropoxy, tert-butoxy, and the like. In addition, alkoxy refers to polyethers such as -O-(CH₂)₂-O-OH₃, and the like.

"Alkanoyl" groups are alkyl linked through a carbonyl, ie, C ₁-C₅-C(O)-. Such groups include formyl, acetyl, propionyl, butyryl, and isobutyryl.

"Acyl" means an alkyl or aryl (Ar) group bonded through a carbonyl group, ie, R-C(O)-. For example, acyl includes a C₁-C₆ alkanoyl, including substituted alkanoyl, wherein the alkyl portion can be substituted by NR⁴R⁵ or a carboxylic or heterocyclic group. Typical acyl groups include acetyl, benzoyl, and the like.

The alkyl, alkenyl, alkoxy, and alkynyl groups described above are optionally substituted, preferably by 1 to 3 groups selected from NR⁴R⁵, phenyl, substituted phenyl, thio C₁-C₆ alkyl, C₁-C₆ alkoxy, hydroxy, carboxy, C₁-C₆ alkoxycarbonyl, halo, nitrile, cycloalkyl, and a 5- or 6-membered carbocyclic ring or heterocyclic ring having I or 2 heteroatoms selected from nitrogen, substituted nitrogen, oxygen, and sulfur. "Substituted nitrogen" means nitrogen bearing C₁-C₆ alkyl or (CH₂)ₙPh where n is 1, 2, or 3. Perhalo and polyhalo substitution is also embraced.

Examples of substituted alkyl groups include 2-aminoethyl, pentachloroethyl, trifluoromethyl, 2-diethylaminoethyl, 2-dimethylaminopropyl, ethoxycarbonylmethyl, 3-phenylbutyl, methanylsulfanylmethyl, methoxymethyl, 3-hydroxypentyl, 2-carboxybutyl, 4-chlorobutyl, 3-cyclopropylpropyl, pentafluoroethyl, 3-morpholinopropyl, piperazinylmethyl, and 2-(4-methylpiperazinyl)ethyl.

Examples of substituted alkynyl groups include 2-methoxyethynyl, 2-ethylsulfanyethynyl, 4-(l-piperazinyl)-3-(butynyl), 3-phenyl-5-hexynyl, 3-diethylamino-3-butynyl, 4-chloro-3-butynyl, 4-cyclobutyl-4-hexenyl, and the like.

Typical substituted alkoxy groups include aminomethoxy, trifluoromethoxy, 2-diethylaminoethoxy, 2-ethoxycarbonylethoxy, 3-hydroxypropoxy, 6-carboxhexyloxy, and the like.

Further, examples of substituted alkyl, alkenyl, and alkynyl groups include dimethylaminomethyl, carboxymethyl, 4-dimethylamino-3-buten-1-yl, 5-ethylmethylamino-3-pentyn-1-yl, 4-morpholinobutyl, 4-tetrahydropyrinidylbutyl, 3-imidazolidin-1-ylpropyl, 4-tetrahydrothiazol-3-ylbutyl, phenylmethyl, 3-chlorophenylmethyl, and the like.

The terms "Ar" and "aryl" refer to unsubstituted and substituted aromatic groups. Heteroaryl groups have from 4 to 9 ring atoms, from 1 to 4 of which are independently selected from the group consisting of O, S, and N. Preferred heteroaryl groups have 1 or 2 heteroatoms in a 5- or 6-membered aromatic ring. Mono and bicyclic aromatic ring systems are included in the definition of aryl and heteroaryl. Typical aryl and heteroaryl groups include phenyl, 3-chlorophenyl, 2,6-dibromophenyl, pyridyl, 3-methylpyridyl, benzothienyl, 2,4,6-tribromophenyl, 4-ethylbenzothienyl, furanyl, 3,4-diethylfuranyl, naphthyl, 4,7-dichloronaphthyl, pyrrole, pyrazole, imidazole, thiazole, and the like.

Preferred Ar groups are phenyl and phenyl substituted by 1, 2, or 3 groups independently selected from the group consisting of alkyl, alkoxy, thio, thioalkyl, hydroxy, -COOR⁷, amino of the formula -NR⁴R⁵, and T(CH₂)ₘQR⁴ or T(CH₂)ₘCO₂R⁴ wherein m is 1 to 6, T is O, S, NR⁴, N(O)R⁴, NR⁴R⁶Y, or CR⁴R⁵, Q is O, S, NR⁵, N(O)R⁵, or NR⁵R⁶Y wherein R⁴ and R⁵ are as described above, and R⁷ is alkyl or substituted alkyl, for example, methyl, trichloroethyl, diphenylmethyl, and the like. The alkyl and alkoxy groups can be substituted as defined above. For example, typical groups are carboxyalkyl, alkoxycarbonylalkyl, hydroxyalkyl, hydroxyalkoxy, and alkoxyalkyl.

The compounds of the present invention can exist in unsolvated forms as well as solvated forms, including hydrated forms. In general, the solvated forms, including hydrated forms, are equivalent to unsolvated forms and are intended to be encompassed within the scope of the present invention.

The compounds of Formula II are capable of further forming both pharmaceutically acceptable formulations comprising salts, including but not limited to acid addition and/or base salts, solvents and N-oxides of a compound of Formula II. This invention also provides pharmaceutical formulations comprising a compound of Formula II together with a pharmaceutically acceptable carrier, diluent, or excipient therefor. All of these forms are within the present invention.

Pharmaceutically acceptable acid addition salts of the compounds of Formula II include salts derived form inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are the salts of amino acids such as arginate, gluconate, galacturonate, and the like; see, for example, Berge, et al., "Pharmaceutical Salts," J. of Pharmaceutical Science, 1977;66:1-19.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metal hydroxides, or of organic amines. Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine; see, for example, Berge, et al., supra.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

The compounds of the present invention are useful for treating cancer (for example, leukemia and cancer of the lung breast, prostate, and skin such as melanoma) and other proliferative diseases including but not limited to psoriasis, HSV, HIV, restenosis, and atherosclerosis. To utilize a compound of the present invention to treat cancer, a patient having cancer is administered a therapeutically effective amount of a pharmaceutically acceptable composition comprising an invention compound.

A further embodiment of this invention is a method of treating subjects suffering from diseases caused by vascular smooth muscle cell proliferation. Compounds within the scope of the present invention effectively inhibit vascular smooth muscle cell proliferation and migration. The method entails inhibiting vascular smooth muscle proliferation, and/or migration by administering an effective amount of a compound of Formula II to a subject in need of treatment.

The compounds of the present invention can be formulated and administered in a wide variety of oral and parenteral dosage forms, including transdermal and rectal administration. It will be recognized to those skilled in the art that the following dosage forms may comprise as the active component, either a compound of Formula II or a corresponding pharmaceutically acceptable salt or solvate of a compound of Formula II.

A further embodiment of this invention is a pharmaceutical formulation comprising a compound of Formula II together with a pharmaceutically acceptable carrier, diluent, or excipient therefor. For preparing pharmaceutical compositions with the compounds of the present invention, pharmacuetically acceptable carriers can be either a solid or liquid. Solid form preparations include powders, tablets, pills, capsules, cachets, suppositories, and dispensible granules. A solid carrier can be one or more substances which may also act as diluents, flavoring agents, binders, preservatives, tablet disintegrating agents, or an encapsulating material.

In powders, the carrier is a finely divided solid such as talc or starch which is in a mixture with the finely divided active component. In tablets, the active component is mixed with the carrier having the necessary binding properties in suitable proportions and compacted in the shape and size desired.

The formulations of this invention preferably contain from about 5% to about 70% or more of the active compound. Suitable carriers include magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. A preferred form for oral use are capsules, which include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogenously therein, as by stirring. The molten homogenous mixture is then poured into convenient size molds, allowed to cool, and thereby to solidify.

Liquid form preparations include solutions, suspensions, and emulsions such as water or water/propylene glycol solutions. For parenteral injection, liquid preparations can be formulated in solution in aqueous polyethylene glycol solution, isotonic saline, 5% aqueous glucose, and the like. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water and mixing with a viscous material, such as natural or synthetic gums, resins, methylcellulose, sodium carboxymethylcellulose, or other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like. Waxes, polymers, microparticles, and the like can be utilized to prepare sustained-release dosage forms. Also, osmotic pumps can be employed to deliver the active compound uniformally over a prolonged period.

The pharmaceutical preparations of the invention are preferably in unit dosage form. In such form, the preparation is subdivided into unit doses containing appropriate quantities of the active component. The unit dosage form can be a packaged preparation, the package containing discrete quantities of preparation, such as packeted tablets, capsules, and powders in vials or ampules. Also, the unit dosage form can be a capsule, tablet, cachet, or lozenge itself, or it can be the appropriate number of any of these in packaged form.

The therapeutically effective dose of a compound of Formula II will generally be from about 1 mg to about 100 mg/kg of body weight per day. Typical adult doses will be about 50 mg to about 800 mg per day. The quantity of active component in a unit dose preparation may be varied or adjusted from about 0.1 mg to about 500 mg, preferably about 0.5 mg to 100 mg according to the particular application and the.potency of the active component. The composition can, if desired, also contain other compatible therapeutic agents. A subject in need of treatment with a compound of Formula I and/or II is administered a dosage of about 1 to about 500 mg per day, either singly or in multiple doses over a 24-hour period.

The compounds of the present invention are capable of binding to and inhibiting the activity of proteins having the ability to phosphorylate other proteins, such as cdks, PDGFr, FGFr, c-src, and EGFr-FL. Cdks form complexes with cyclins, and these complexes phosphorylate key proteins allowing cells to proceed through the cell cycle (Meijer L., Progress in Cell Cycle. Research, 1995;1:351-363). The compounds of this invention inhibit this phosphorylation and therefore can be used as anti-proliferative agents for the treatment of cancer and/or restenosis and other proliferative diseases.

Because of their inhibitory activity against cdks and other kinases, the compounds of the present invention are also useful research tools for studying the mechanism of action of those kinases, both in vitro and in vivo.

While the forms of the invention herein constitute presently preferred embodiments, many others are possible. It is not intended herein to mention all of the possible equivalent forms or ramifications of the invention. It is understood that the terms used herein are merely descriptive rather than limiting, and those skilled in the art will realize that various changes may be made without departing from the spirit or scope of the invention.

The following compounds illustrate specific embodiments provided by the present invention, and the compounds listed below are among the preferred embodiments.

Compounds of Formula II may be prepared according to the syntheses outlined in Schemes 1 through 9, *Infra.* Although these schemes often indicate exact structures, those with ordinary skill in the art will appreciate that the methods apply widely to analogous compounds of Formula II, given appropriate consideration to protection and deprotection or reactive functional groups by methods standard to the art of organic chemistry. For example, hydroxy groups, in order to prevent unwanted side reactions, generally need to be converted to ethers or esters during chemical reactions at other sites in the molecule. The hydroxy protecting group is readily removed to provide the free hydroxy group. Amino groups and carboxylic acid groups are similarly derivatized to protect them against unwanted side reactions. Typical protecting groups and methods for attaching and cleaving them are described fully by Greene and Wuts in Protective Groups In Organic Synthesis, John Wiley and Sons, New York, (2nd Ed., 1991), and McOmie, Protective Groups in Organic Chemistry, Pleura Press, New York, 1973.

Scheme 1 describes a typical method for the preparation of the pyrido[2,3-d]pyrimidin-7(8H)-ones. The synthesis begins with commercially available (Aldrich) 4-chloro-2-methylthio-pyrimidine-5-carboxylic acid ethyl ester. Displacement of the 4-chloro group with an amine in a solvent such as tetrahydrofuran in the presence or absence of a tertiary amine such as triethylamine provides the corresponding 4-amino-2-methylthio-pyrimidine-5-carboxylic acid ethyl ester. The amine used can be anhydrous or in an aqueous solution as with methyl or ethyl amine. The use of aqueous ammonium hydroxide provides the corresponding primary amine at position 4. Oxidation of the methylthio group with an oxidant such as an oxaziridine in a solvent such as chloroform at room temperature provides the methyl sulfoxide derivative. Displacement of the sulfoxide with an amine results in formation of the corresponding 2,4-diamino-pyrimidine-5-carboxylic acid ethyl ester. The temperature required for the displacement depends upon the amine used. Aromatic, secondary, and tertiary amines usually require higher temperatures than primary aliphatic or benzyl amines. When aromatic amines such as aniline are used, the reaction is usually run with the amine as the solvent at high temperatures. The ester group is sequentially reduced to the alcohol, preferably with lithium aluminum hydride in tetrahydrofuran, and then oxidized to the aldehyde. While sodium dichromate can be used as the oxidant, superior results are obtained with manganese II oxide in chloroform.

The 2,4-di-amino-pyrimidine-5-carboxaldehydes can be reacted with either a stabilized phosphorane, a phosphonate ester in the presence of a base, or any alternative Wittig or Horner-Emmons reagent to provide the corresponding unsaturated ester. The resulting double bond can be trans, cis, or a mixture of both. For example, reaction of a 2,4-diamino-pyrimidine-5-carboxaldehyde with an excess amount of the stabilized phosphorane (carbethoxymethylene)triphenylphosphorane in tetrahydrofuran at reflux temperature gives mainly, or in some cases exclusively, the trans unsaturated ethyl ester. Upon treatment with base, ring closure occurs to give the desired pyrido[2,3-d]pyrimidin-7(8H)-one. This reaction can be carried out using a tertiary amine such as triethylamine or, preferably, N,N-diisopropylethyl amine as the solvent, with 1 to 10 equivalents of 1,8-diazabicyclo[5.4.0]undec-7-ene present. The reaction is carried out at elevated temperature, and is usually complete in 2 to 24 hours. Alternatively, the 2,4-diamino-pyrimidine-5-carboxaldehyde can be reacted with a phosphonate ester such as bis(2,2,2-trifluoroethyl)(methoxycarbonyl-methyl)-phosphonate using a strongly dissociated base (Tetrahedron Lett., 1983:4405) to give predominately, if not exclusively, the cis unsaturated ester. Upon treatment with base under the conditions discussed previously, ring closure occurs.

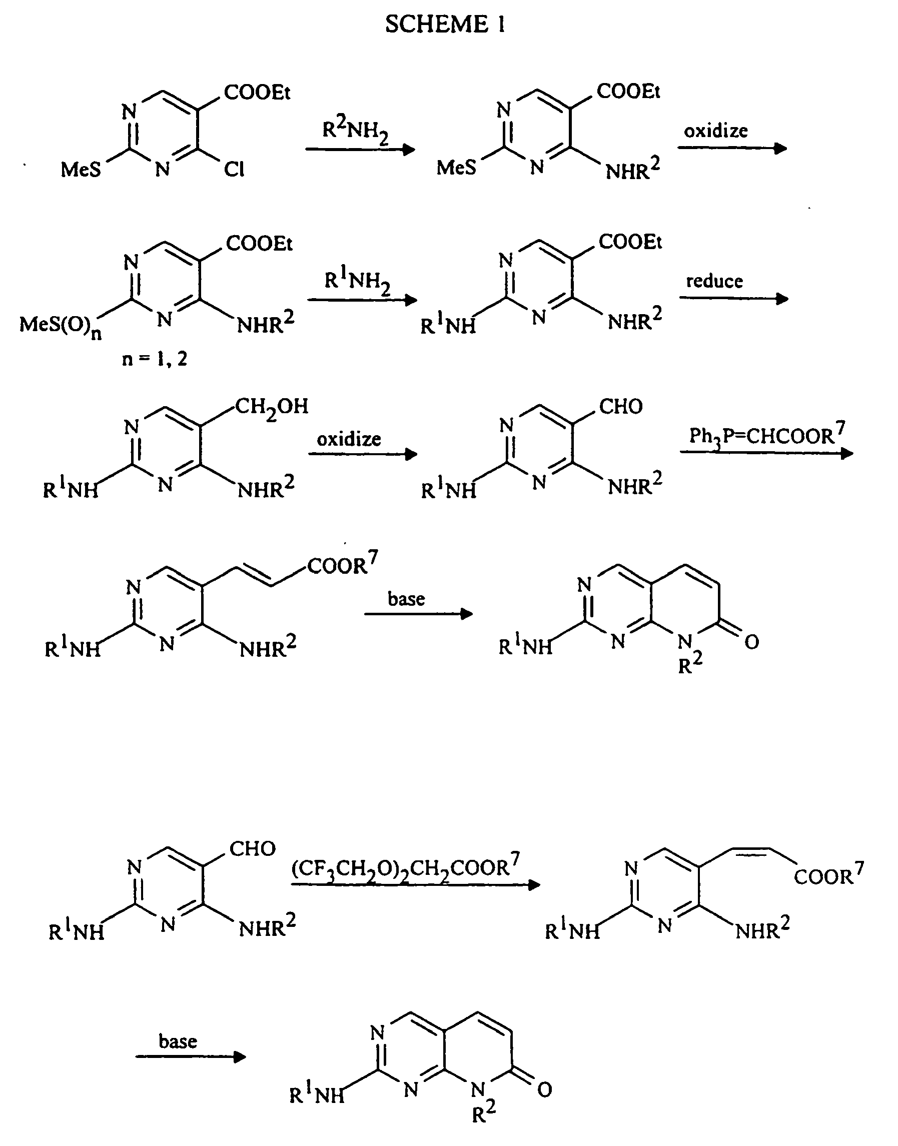

Scheme 2 depicts the preparation of pyrido[2,3-d]pyrimidin-7(8H)-ones where R² is H. The sequence of reactions is the same as Scheme 1, where the initial step uses ammonium hydroxide giving the 4-primary amino pyrimidine. The resultant pyrido[2,3-d]pyrimidin-7(8H)-ones where R² is equal to H can be alkylated at the 8-position by treatment with a base such as sodium hydride in a solvent such as dimethylformamide or tetrahydrofuran at temperatures ranging from 40°C to reflux, thus providing the corresponding pyrido[2,3-d]pyrimidin-7(8H)-ones where R² is other than H. The advantage of the route shown in Scheme 2 is that it allows for several R² analogs to be prepared from a common intermediate. The required aldehyde can also be obtained by reduction of the corresponding nitrile (J. Org. Chem., 1960;82:5711) with a reducing agent, preferably diisobutylaluminum hydride.

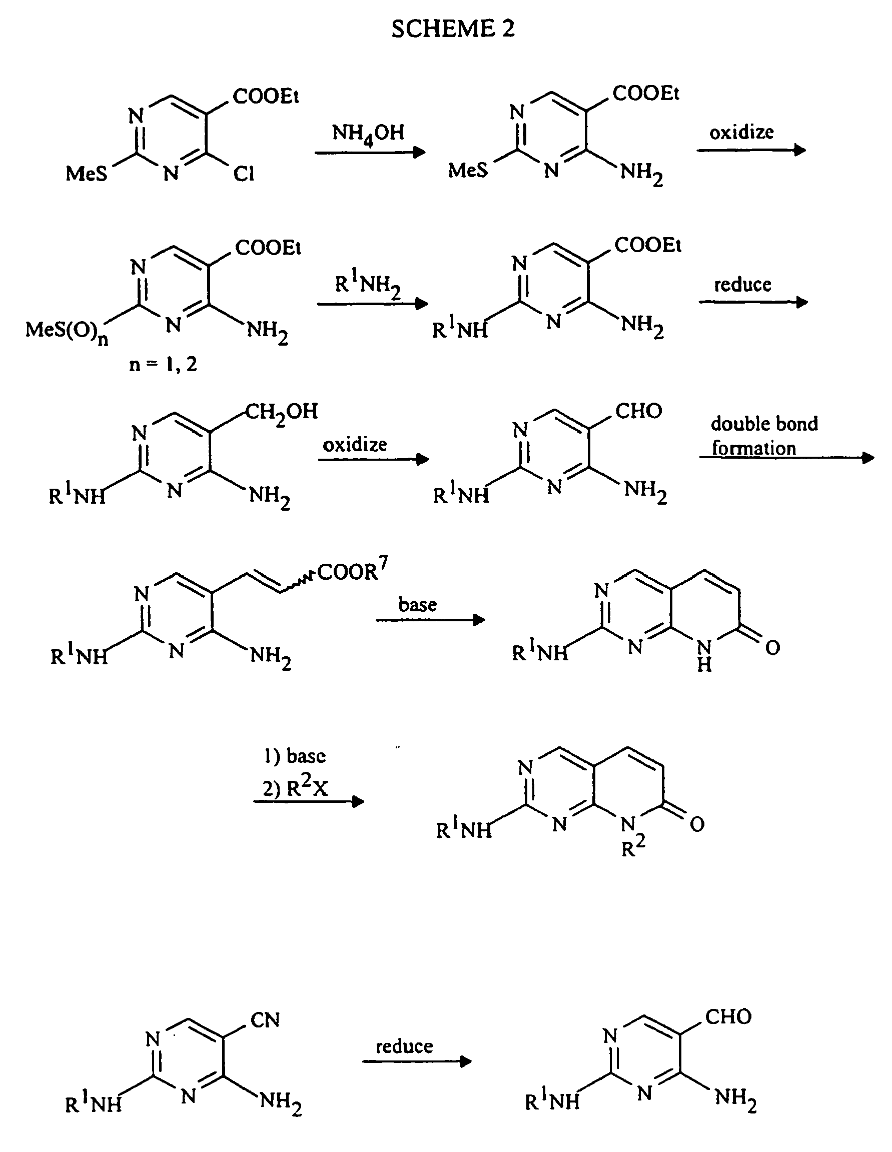

A route that allows for the preparation of several analogs with various R¹ groups from a common intermediate is shown in Scheme 3. The initial step is the same as in Scheme 1, but instead of oxidizing the methyl thio group, the ester is sequentially reduced and then oxidized using the conditions described in Scheme 1 to provide the corresponding 2-methylthio-4-amino-pyrimidine-5-carboxaldehyde. This aldehyde is converted to the corresponding unsaturated ester using the conditions described in Scheme 1. The methylthio group can be displaced directly with primary alkyl amines to give the pyrido[2,3-d]pyimidin-7-(8H)-ones of the invention where R¹ is H or a primary alkyl group. The methylthio group can also be converted to the corresponding sulfoxide by treatment with an oxidizing agent, preferably an oxaziridine, in a solvent such as chloroform at room temperature. Alternatively, an oxidizing agent, such as m-chloroperbenzoic acid, can be used in excess to convert the methylthio derivative to the corresponding methyl sulfone. Upon treatment of these oxidized derivatives with an amine, usually with several equivalents of the amine at elevated temperatures in the case of aromatic or tertiary amines, Pyrido[2,3-d]pyrimidin-7(8H)-ones with various R¹ groups are obtained. In some cases a solvent such as tetrahydrofuran or dimethylsulfoxide can be used.

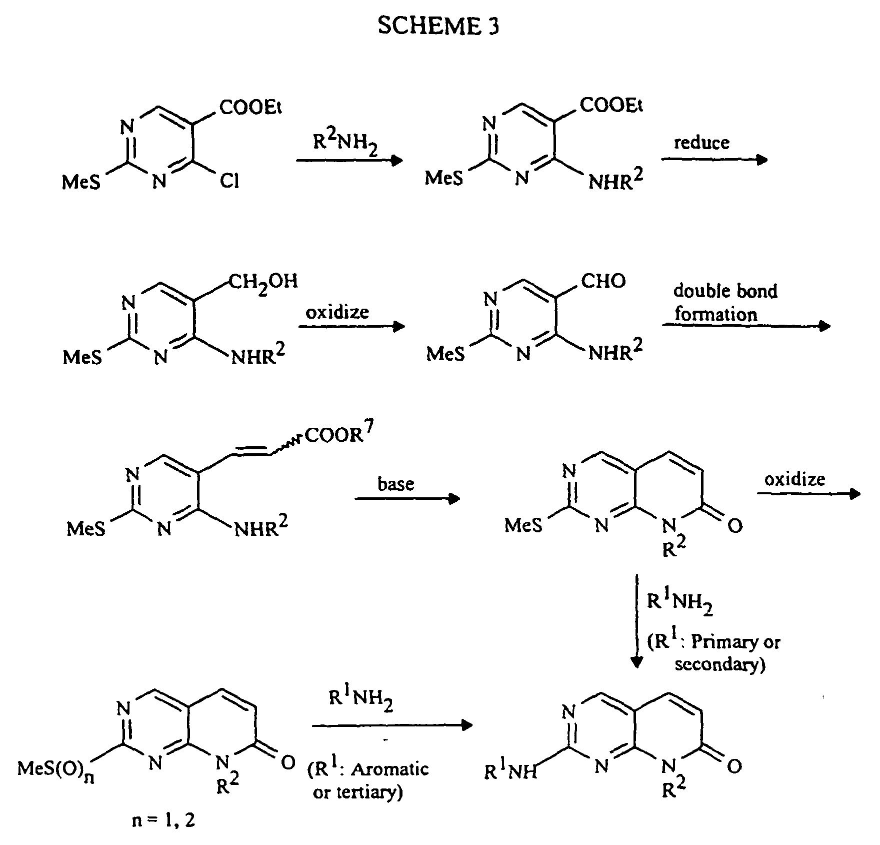

The most convergent route to the compounds of the invention where X is O is via the synthesis of 2-methanesulfanyl-8H-pyrido[2,3-d]pyrimidin-7-one which is depicted in Scheme 4. This key intermediate is prepared by the methods discussed in the previous schemes and is converted to the compounds of the invention by 2 routes, shown in Scheme 5. In the first, the methylthio group is converted to an amino group, in some cases via an oxidized intermediate. These derivatives are then alkylated at N8 to give the desired compounds. Alternatively, 2-methanesulfanyl-8H-pyrido[2,3-d]pyrimidin-7-one is first alkylated at N8, then the methylthio group, or an oxidized derivative, is displaced by an amine.

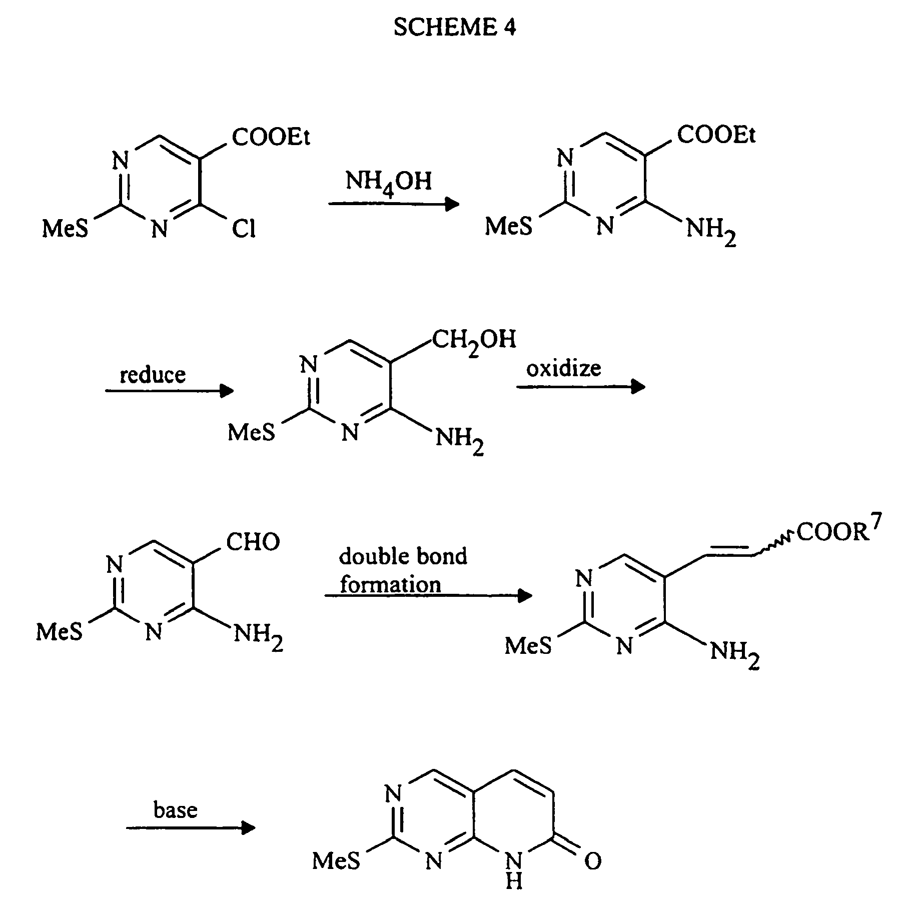

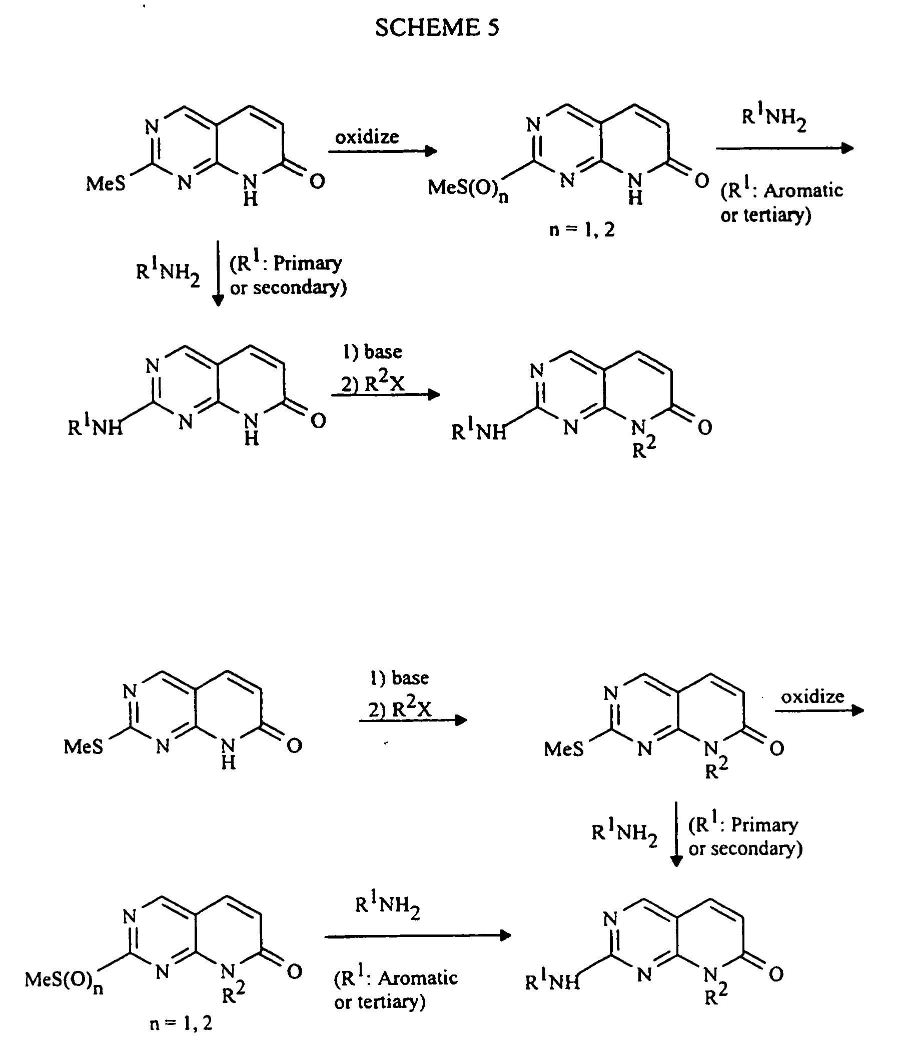

Scheme 6 describes a typical method for the preparation of the pyrido[2,3-d]pyrimidin-7(8H)-imines (X = NH). The synthesis begins with the 2,4-diamino-pyrimidine-5-carboxaldehyde previously described in Scheme 1. Reaction with diethyl cyanomethylphosphonate in the presence of a base, such as sodium hydride, in a solvent such as tetrahydrofuran, provides the corresponding unsaturated nitrile. This nitrile is then cyclized to give the pyrido[2,3-d]pyrimidin-7(8H)-imine under the same conditions used to prepare the pyrido[2,3-d]pyrimidin-7(8H)-ones of Scheme 1. Alternatively, the pyrimidine-5-carboxaldehyde can contain a methylthio group at C₂. After formation of the unsaturated nitrile followed by ring closure, the methylthio group at C₂ can be converted to an amino group by the methology previously mentioned. The pyrido[2,3-d]pyrimidin-7(8H)-imines can also be converted to the pyrido[2,3-d]pyrimidin-7(8H)-ones by direct hydrolysis with concentrated acid, such as hydrochloric acid, at elevated temperatures. A milder method can also be used where the imine is first acylated with acetic anhydride. The hydrolysis of this acyl intermediate to the 7-one occurs under shorter reaction time and lower reaction temperatures.

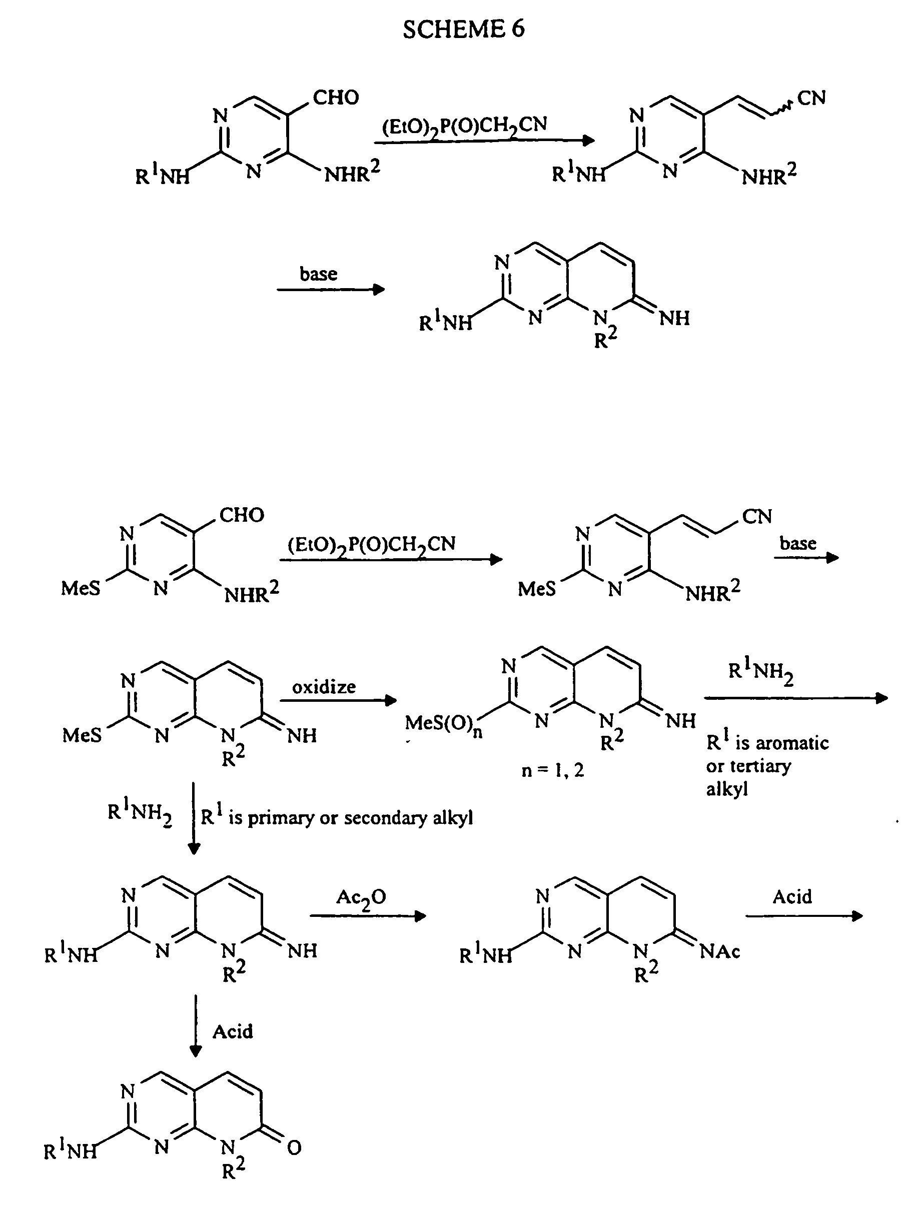

As shown in Scheme 7, those compounds where there is no double bond between C₅ and C₆ can be prepared by direct reduction of the double bond for those cases where X is O. Alternatively, a more preferred route is to reduce the double bond of the precursor unsaturated ester. This can be accomplished with a metal catalyst, such as palladium, in the presence of hydrogen under pressure. This saturated ester is then cyclized using the conditions discussed previously. Due to the propensity of the imine or nitrile group to be reduced under the conditions used to reduce the carbon-carbon double bond, a different route is required to prepare the compounds of the invention without a double bond at C₅-C₆ for those cases where X is NH. The saturated ester is hydrolyzed to the acid and then converted to the primary amide, by activation of the carboxylate with an acid chloride or N,N-carbonyldiimidazole, followed by treatment with ammonia gas or aqueous ammonium hydroxide. The primary amide is dehydrated to the corresponding nitrile with a reagent such as phosphorous pentoxide. This saturated nitrile is then cyclized using the conditions described previously.

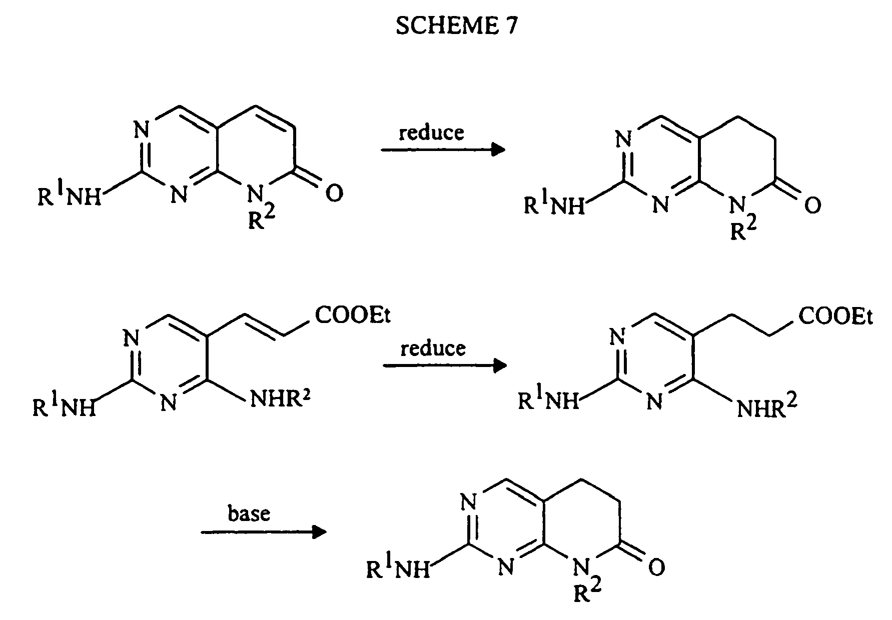

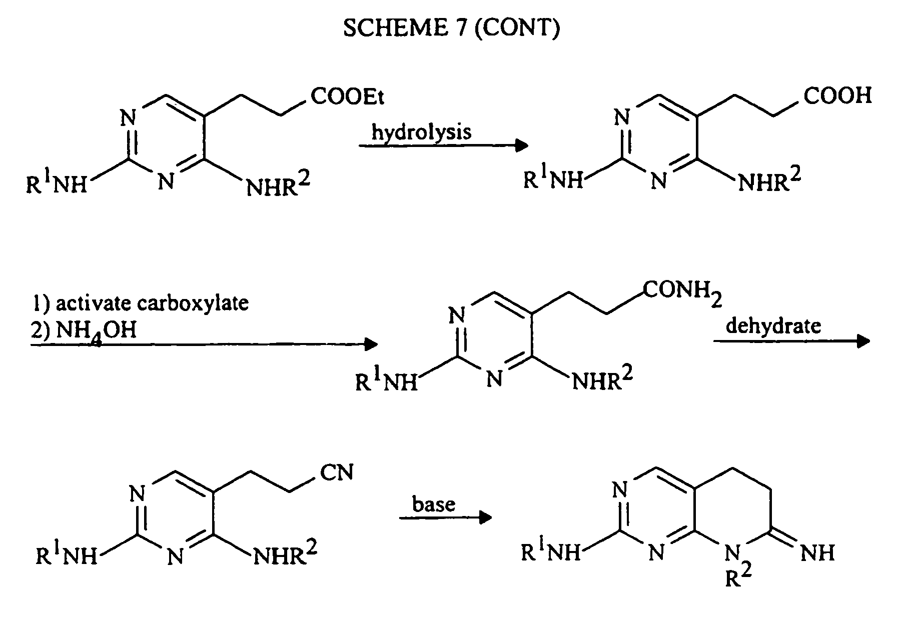

It should be noted that while the routes depicted in the earlier schemes showed the preparation of the pyrido[2,3-d]pyrimidin-7(8H)-ones where R³ is H, these routes can be readily modified to prepare compounds where R³ is lower alkyl, as shown in Scheme 8. Treatment with base provides compounds of the invention where X is O and R³ is lower alkyl. Alternatively, these same reactions can be carried out on the 2-methylthio-4-amino-pyrimidine 5-carboxaldehyde and, after cyclization, the 2-methylthio group can be converted to the corresponding amine. Suitable modification of Scheme 6 would lead to the preparation of the pyrido[2,3-d]pyrimidin-7(8H)-imines where R³ is lower alkyl.

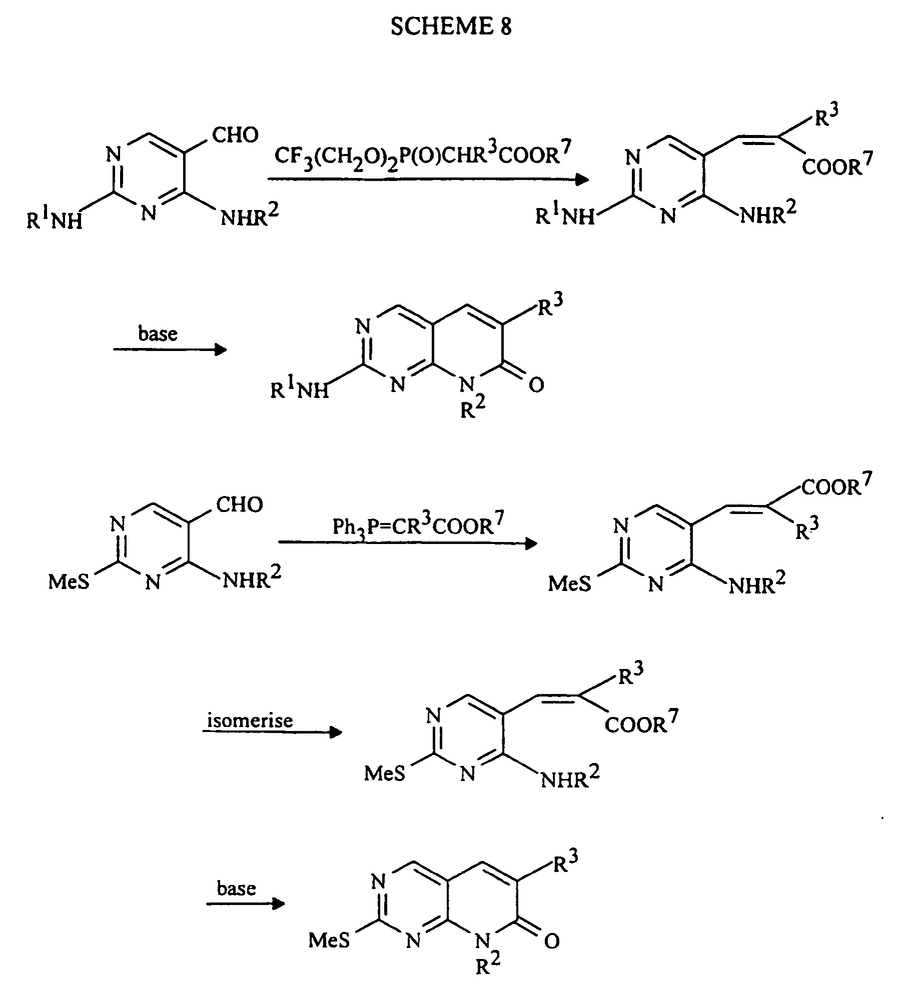

Additional 2,4-diaminopyrimidines of the invention can be prepared as shown in Scheme 9. For example, those analogs where Z is CH₂OH are prepared by reduction of the ester with a reducing agent such as an excess of diisobutylaluminum hydride in a solvent such as tetrahydrofuran or chloroform. Subsequent oxidation with an oxidizing agent such as manganese oxide, or Swern's conditions, provides the compound where Z is CHO. Compounds where Z is COOR⁷ or CONHR⁷ can be obtained from the compound where Z is COOH. Activation of the carboxylate with an acid chloride or 1,1-carbonyldiimidazole, followed by addition of an alcohol of formula R⁷OH or an amine of formula R⁷NH₂, would provide those compounds where Z is COOR⁷ and CONHR⁷, respectively.

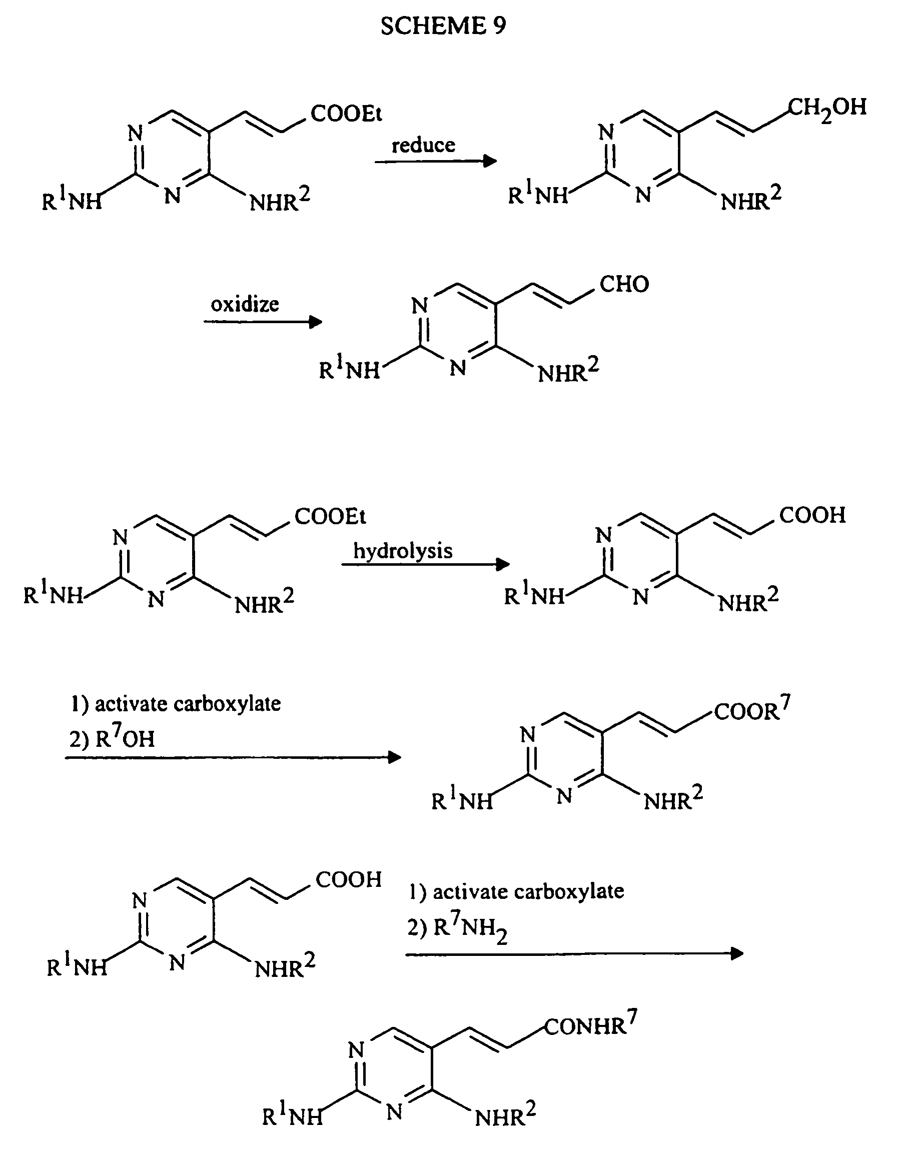

### EXAMPLES

The following examples are for illustrative purposes only and are not intended, nor should they be construed as limiting the invention in any manner. Those skilled in the art will appreciate that variations and modifications can be made without violating the spirit or scope of the invention.

### EXAMPLE 1

### 4-Ethylamino-2-methanesulfanyl-pyrimidine-5-carboxylic acid ethyl ester

To a room temperature solution of 4-chloro-2-methanesulfanyl-pyrimidine-5-carboxylic acid ethyl ester (10.00 g , 43.10 mmol) in 150 mL of tetrahydrofuran was added triethylamine (18.5 mL, 133 mmol) followed by 9 mL of a 70% aqueous solution of ethylamine. The solution was stirred for 30 minutes then concentrated in vacuo and partitioned between chloroform and saturated aqueous sodium bicarbonate. The organic layer was dried over magnesium sulfate, filtered, and concentrated to provide 9.32 g (90%) of 4-ethylamino-2-methanesulfanyl-pyrimidine-5-carboxylic acid ethyl ester as an oil.

| Analysis calculated for C₁₀H₁₅N₃O₂S: | | | |
|---|---|---|---|
| | C, 49.77; | H, 6.27; | N, 17.41. |
| Found: | C, 49.77; | H, 6.24; | N, 17.30. |

### EXAMPLE 2

### (4-Ethylamino-2-methanesulfanyl-pyrimidin-5-yl)-methanol

A solution of 4-ethylamino-2-methanesulfanyl-pyrimidine-5-carboxylic acid ethyl ester (8.93 g, 37.1 mmol) in 100 mL of tetrahydrofuran was added dropwise to a room temperature suspension of lithium aluminum hydride (2.30 g, 60.5 mmol) in 100 mL of tetrahydrofuran. After 10 minutes, the reaction was carefully quenched with 4.5 mL of water, 4.5 mL of 15% NaOH, and an additional 16 mL of water, and the mixture was stirred for 1.5 hours. The white precipitate was removed by filtration, washing with ethyl acetate. The filtrate was concentrated in vacuo and 1:1 hexane:ethyl acetate was added. The solids were collected to give 6.77 g (92%) of (4-ethylamino-2-methanesulfanyl-pyrimidin-5-yl)-methanol, mp 152-156°C.

| Analysis calculated for C₈H₁₃N₃OS: | | | |
|---|---|---|---|
| | C, 48.22; | H, 6.58; | N, 21.09. |
| Found: | C, 48.14; | H, 6.61; | N, 20.85. |

### EXAMPLE 3

### 4-Ethylamino-2-methanesulfanyl-pyrimidine-5-carboxaldehyde

To (4-ethylamino-2-methanesulfanyl-pyrimidin-5-yl)-methanol (6.44 g, 32.4 mmol) in 600 mL of chloroform was added manganese oxide (21.0 g, 241 mmol). The suspension was stirred at room temperature for 2 hours and an additional 5.5 g of manganese oxide was added. Stirring was continued for 4.5 hours. The mixture was filtered through celite, washing with chloroform. The filtrate was concentrated in vacuo to give 6.25 g (97%) of 4-ethylamino-2-methanesulfanyl-pyrimidine-5-carboxaldehyde, mp 58-61°C.

| Analysis calculated for C₈H₁₁N₃OS: | | | |
|---|---|---|---|
| | C, 48.71; | H, 5.62; | N; 21.30. |
| Found: | C, 48.62; | H, 5.60; | N, 21.28. |

### EXAMPLE 4

### 4-Ethylamino-2-methanesulfinyl-pyrimidine-5-carboxylic acid ethyl ester

To a room temperature solution of 4-ethylamino-2-methanesulfanyl-5-pyrimidinecarboxylate ethyl ester (2.011 g , 8.34 mmol) in 70 mL of chloroform was added (±)-*trans*-2-(phenylsulfonyl)-3-phenyloxaziridine (2.70 g, 10.34 mmol). The solution was stirred at room temperature for 7 hours then concentrated in vacuo. The residue was purified by flash chromatography, eluting with a gradient of ethyl acetate to 3% methanol in ethyl acetate, to provide 2.07 g (97%) of 4-ethylamino-2-methanesulfinyl-pyrimidine-5-carboxylic acid ethyl ester, mp 54-56°C.

| Analysis calculated for C₁₀H₁₅N₃O₃S: | | | |
|---|---|---|---|
| | C, 46.68; | H, 5.88; | N, 16.33. |
| Found: | C, 46.56; | H, 5.68; | N, 16.23. |

### EXAMPLE 5

### 4-Ethylamino-2-phenylamino-pyrimidine-5-carboxylic acid ethyl ester

A solution of 4-ethylamino-2-methanesulfinyl-pyrimidine-5-carboxylic acid ethyl ester (5.38 g, 20.9 mmol) in 4 mL of aniline was heated at 130°C for 1 hour. The solution was cooled to room temperature, and 20 mL of 1:1 hexane:ethyl acetate was added. The resultant white solid was collected by filtration to give 1.96 g (33%) of the title product. The filtrate was concentrated in vacuo and purified by flash chromatography eluting with 3:1 hexane:ethyl acetate to provide an additional 257 mg (4%) of pure 4-ethylamino-2-phenylamino-pyrimidine-5-carboxylic acid ethyl ester, mp 145-147°C.

| Analysis calculated for C₁₅H₁₈N₄O₂: | | | |
|---|---|---|---|
| | C, 62.92; | H, 6.34; | N, 19.57. |
| Found: | C, 62.83; | H, 6.24; | N, 19.50. |

### EXAMPLE 6

### (4-Ethylamino-2-phenylamino-pyrimidin-5-yl)-methanol

A solution of 4-ethylamino-2-phenylamino-pyrimidine-5-carboxylic acid ethyl ester (109 mg, 0.38 mmol) in 6 mL of tetrahydrofuran was added dropwise to a room temperature suspension of lithium aluminum hydride (35 mg, 0.92 mmol) in 5 mL of tetrahydrofuran. After 25 minutes, an additional 30 mg of lithium aluminum hydride was added, and stirring was continued for 30 minutes. The reaction was carefully quenched with 120 µL of water, 200 µL of 15% NaOH, and an additional 300 µL of water. After stirring for 1 hour, the white precipitate was removed by filtration, washing with ethyl acetate. The filtrate was concentrated in vacuo, and the crude material was purified by flash chromatography eluting with ethyl acetate to provide 36 mg (39%) of (4-ethylamino-2-phenylamino-pyrimidin-5-yl)-methanol, mp 174-176°C.

| Analysis calculated for C₁₃H₁₆N₄O: | | | |
|---|---|---|---|
| | C, 63.92; | H, 6.60; | N, 22.93. |
| Found: | C, 63.97; | H, 6.58; | N, 22.79. |

### EXAMPLE 7

### 4-Ethylamino-2-phenylamino-pyrimidine-5-carboxaldehyde

To a solution of (4-ethylamino-2-phenylamino-pyrimidin-5-yl)-methanol (173 mg, 0.71 mmol) in 15 mL of chloroform was added manganese oxide (600 mg, 6.89 mmol). After stirring at room temperature overnight, the mixture was filtered through a pad of celite, washing with chloroform. The filtrate was concentrated in vacuo to give 170 mg (99%) of 4-ethylamino-2-phenylamino-pyrimidine-5-carboxaldehyde, mp 155-157°C.

| Analysis calculated for C₁₃H₁₄N₄O: | | | |
|---|---|---|---|
| | C, 64.45; | H, 5.82; | N, 23.12. |
| Found: | C, 64.31; | H, 6.01; | N, 22.98. |

### EXAMPLE 8

### 4-Methylamino-2-methanesulfanyl-pyrimidine-5-carboxylic acid ethyl ester

To a room temperature solution of 4-chloro-2-methanesulfanyl-pyrimidine-5-carboxylic acid ethyl ester (18.66 g , 80.4 mmol) in 260 mL of tetrahydrofuran was added triethylamine (34 mL, 244 mmol) followed by 30 mL of a 40% aqueous solution of methylamine. The solution was stirred for 30 minutes, then was concentrated in vacuo and partitioned between chloroform and saturated aqueous sodium bicarbonate. The organic layer was washed with brine, dried over magnesium sulfate, filtered, and concentrated to provide a white solid. The solid was suspended in hexane and filtered to provide 14.70 g (81 %) of 4-methylamino-2-methanesulfanyl-pyrimidine-5-carboxylic acid ethyl ester, mp 91-93°C. Literature mp 93-94°C: J. Org. Chem., 1960:2137.

| Analysis calculated for C₉H₁₃N₃O₂S: | | | |
|---|---|---|---|
| | C, 47.56; | H, 5.76; | N, 18.49. |
| Found: | C, 47.93; | H, 5.67; | N, 18.58. |

### EXAMPLE 9

### (4-Methylamino-2-methanesulfanyl-pyrimidin-5-yl)-methanol

A solution of 4-methylamino-2-methanesulfanyl-pyrimidine-5-carboxylic acid ethyl ester (4.36 g, 19.3 mmol) in 60 mL of tetrahydrofuran was added dropwise to a room temperature suspension of lithium aluminum hydride (1.10 g, 29.0 mmol) in 40 mL of tetrahydrofuran. After 10 minutes, the reaction was carefully quenched with 2 mL of water, 2 mL of 15% NaOH, and 7 mL of water, and the mixture was stirred for 1 hour. The white precipitate was removed by filtration, washing with ethyl acetate. The filtrate was concentrated in vacuo and 25 mL of 3:1 hexane:ethyl acetate was added. The solids were collected to give 2.99 g (84%) of (4-methylamino-2-methanesulfanyl-pyrimidin-5-yl) methanol, mp 155-157°C. Literature, mp 157-159°C: J. Chem. Soc., 1968:733.

| Analysis calculated for C₇H₁₁N₃OS : | | | |
|---|---|---|---|
| | C, 45.39; | H, 5.99; | N, 22.68. |
| Found: | C, 45.42; | H, 5.93; | N, 22.42. |

### EXAMPLE 10

### 4-Methylamino-2-methanesulfanyl-pyrimidine5-carboxaldehyde

To (4-methylamino-2-methanesulfanyl-pyrimidin-5-yl)-methanol (5.78 g, 31.2 mmol) in 600 mL of chloroform was added manganese oxide (25.0 g, 286 mmol). The suspension was stirred at room temperature for 6 hours then filtered through celite washing with 300 mL of chloroform. The filtrate was concentrated in vacuo, and hexane was added to the residue. The solid was collected to give 5.35 g (93%) of 4-methylamino-2-methanesulfanyl-pyrimidine-5-carboxaldehyde, mp 97-100°C.

### EXAMPLE 11

### 4-Amino-2-methanesulfanyl-pyrimidine-5-carboxylic acid ethyl ester

To a room temperature solution of 4-chloro-2-methanesulfanyl-pyrimidine-5-carboxylic acid ethyl ester (15.0 g , 65 mmol) in 200 mL of tetrahydrofuran was added 25 mL of triethylamine followed by 35 mL of aqueous ammonium hydroxide. After stirring at room temperature for 1.5 hours, an additional 30 mL of aqueous ammonium hydroxide was added, and stirring was continued for 1 hour. The reaction mixture was concentrated in vacuo and partitioned between ethyl acetate and saturated aqueous sodium bicarbonate. The organic layer was washed with brine, dried over magnesium sulfate, filtered, and concentrated in vacuo. Ethyl acetate and hexane were added, and the resultant solid was collected by filtration to provide 10.84 g (79%) of 4-amino-2-methanesulfanyl-pyrimidine-5-carboxylic acid ethyl ester.

### EXAMPLE 12

### (4-Amino-2-methanesulfanyl-pyrimidin-5-yl)-methanol

A solution of 4-amino-2-methanesulfanyl-pyrimidine-5-carboxylic acid ethyl ester (13.36 g, 63 mmol) in 250 mL of tetrahydrofuran was added dropwise to a room temperature suspension of lithium aluminum hydride (3.82 g, 100 mmol) in 250 mL of tetrahydrofuran. After 30 minutes, the reaction was cooled to 0°C, and isopropyl alcohol was added until bubbling diminished. The reaction was quenched with 15 mL of water, 15 mL of 15% NaOH, and 50 mL of water, and the mixture was stirred for 1 hour. The white precipitate was removed by filtration, washing with ethyl acetate. The filtrate was concentrated in vacuo and 3:1 hexane:ethyl acetate was added. The solids were collected, washed with 3:1 hexane:ethyl acetate, followed by hexane. The solid was dissolved in ethyl acetate, and the solution was dried over magnesium sulfate. Filtration followed by concentration in vacuo gave 8.14 g (76%) of (4-amino-2-methanesulfanyl-pyrimidin-5-yl)-methanol.

| Analysis calculated for C₆H₉N₃OS: | | | |
|---|---|---|---|
| | C, 42.09; | H, 5.30; | N, 24.54. |
| Found: | C, 42.31; | H, 5.24; | N, 24.27. |

### EXAMPLE 13

### 4-Amino-2-methanesulfanyl-pyrimidine-5-carboxaldehyde

To (4-amino-2-methanesulfanyl-pyrimidin-5-yl)-methanol (8.14 g, 48 mmol) in 1 L of chloroform was added manganese oxide (33.13 g, 381 mmol). The suspension was stirred at room temperature overnight then filtered through celite washing with 300 mL of chloroform. The filtrate was concentrated in vacuo to give 8.14 g (quantitative yield) of 4-amino-2-methanesulfanyl-pyrimidine-5-carboxaldehyde, mp 185-187°C. Literature mp = 183-184°C, JOC, 1958;23:1738.

| Analysis calculated for C₆H₇N₃OS: | | | |
|---|---|---|---|
| | C, 42.59; | H, 4.17; | N, 24.83. |
| Found: | C, 42.84; | H, 4.21; | N, 24.73. |

### EXAMPLE 14

### 4-(4-Methoxybenzylamino)-2-methanesulfanyl-pyrimidine-5-carboxylic acid ethyl ester

To a room temperature solution of 4-chloro-2-methanesulfanyl-pyrimidine-5-carboxylic acid ethyl ester (6.05 g , 26.07 mmol) in 60 mL of tetrahydrofuran was added triethylamine (11 mL, 79.5 mmol) followed by 3.6 mL (27.6 mmol) of 4-methoxybenzylamine. The solution was stirred for 1 hour then filtered. The white solid was washed with ethyl acetate, and the filtrate was concentrated in vacuo. The residue was partitioned between chloroform and saturated aqueous sodium bicarbonate. The organic layer was dried over magnesium sulfate, filtered, and concentrated to provide 7.60 g (88%) of 4-(4-methoxybenzylamino)-2-methanesulfanyl-pyrimidine-5-carboxylic acid ethyl ester, mp 72-74°C.

| Analysis calculated for C₁₆H₁₉N₃O₃S: | | | |
|---|---|---|---|
| | C, 57.64; | H, 5.74; | N, 12.60. |
| Found: | C, 57.65; | H, 5.80; | N, 12.57. |

### EXAMPLE 15

### [4-(4-Methoxybenzylamino)-2-methanesulfanyl-pyrimidin-5-yl]-methanol

A solution of 4-(4-methoxybenzylamino)-2-methanesulfanyl-pyrimidine-5-carboxylic acid ethyl ester (6.89 g, 20.70 mmol) in 60 mL of tetrahydrofuran was added dropwise to a room temperature suspension of lithium aluminum hydride (1.17 g, 30.8 mmol) in 40 mL of tetrahydrofuran. After 30 minutes, the reaction was carefully quenched with 2 mL of water, 2 mL of 15% NaOH, and 7 mL of water, and the mixture was stirred to give a white precipitate. The solid was removed by filtration, washing with ethyl acetate. The filtrate was partially concentrated in vacuo, and the white solid was collected by filtration to give 1.47 g (24%) of product. The filtrate was concentrated, and upon addition of 3:1 hexane:ethyl acetate, additional solid formed. The precipitate was collected to give 3.16 g (52%) of [4-(4-methoxybenzylamino)-2-methanesulfanyl-pyrimidin-5-yl]-methanol, mp 163-165°C.

| Analysis calculated for C₁₄H₁₇N₃O₂S: | | | |
|---|---|---|---|
| | C, 57.71; | H, 5.88; | N, 14.42. |
| Found: | C, 57.78; | H, 5.88; | N, 14.36. |

### EXAMPLE 16

### 4-(4-Methoxybenzylamino)-2-methanesulfanyl-pyrimidine-5-carboxaldehyde

To [4-(4-methoxybenzylamino)-2-methanesulfanyl-pyrimidin-5-yl]-methanol (4.08 g, 14.02 mmol) in 400 mL of chloroform was added manganese oxide (10.90 g, 125 mmol). The suspension was stirred at room temperature for 8 hours and then filtered through celite washing with chloroform. The filtrate was concentrated in vacuo followed by the addition of hexane to give 3.87 g (96%) of 4-(4-methoxybenzylamino)-2-methanesulfanyl-pyrimidine-5-carboxaldehyde, mp 87-89°C.

| Analysis calculated for C₁₄H₁₅N₃O₂S: | | | |
|---|---|---|---|
| | C, 58.11; | H, 5.23; | N, 14.52. |
| Found: | C, 57.88; | H, 5.12; | N, 14.35. |

### EXAMPLE 17

### Ethyl 3-(4-Ethylamino-2-phenylamino-pyrimidin-5-yl)acrylate

To a room temperature solution of 4-ethylamino-2-phenylamino-pyrimidine-5-carboxaldehyde (320 mg, 1.32 mmol) in 12 mL of tetrahydrofuran was added (carbethoxymethylene)triphenylphosphorane (720 mg, 2.07 mmol). The reaction mixture was heated at reflux for 7 hours then stirred at room temperature overnight. An additional amount of (carbethoxymethylene)triphenylphosphorane (300 mg, 0.86 mmol) was added, and the reaction mixture was heated at reflux for an additional 8 hours then stirred at room temperature for 3 days. The reaction mixture was concentrated in vacuo, and the residue was purified by flash chromatography, eluting with 1:2 ethyl acetate:hexane, to provide 357 mg (86%) of ethyl 3-(4-ethylamino-2-phenylamino-pyrimidin-5-yl)acrylate, mp 125-126°C.

| Analysis calculated for C₁₇H₂₀N₄O₂: | | | |
|---|---|---|---|
| | C, 65.37; | H, 6.45; | N, 17.94. |
| Found: | C, 65.40; | H, 6.57; | N, 17.64. |

### EXAMPLE 19

### Ethyl 3-(4-Amino-2-methanesuvanyl-pyrimidin-5-yl)acrylate

To a room temperature solution of 4-amino-2-methanesulfanyl-pyrimidine-5-carbaldeyde (4.08 g, 24.14 mmol) in 100 mL of tetrahydrofuran was added (carbethoxymethylene)triphenylphosphorane (10.80 g, 31 mmol). The reaction mixture was heated at reflux for 3 hours then stirred at room temperature overnight. The reaction mixture was concentrated in vacuo, and the residue was purified by flash chromatography, eluting with 1:1 ethyl acetate:hexane, to provide 4.30 g (75%) of ethyl 3-(4-amino-2-methanesulfanyl-pyrimidin-5-yl)acrylate, mp softens at 108°C.

| Analysis calculated for C₁₀H₁₃N₃O₂S: | | | |
|---|---|---|---|
| | C, 50.19; | H, 5.48; | N, 17.56. |
| Found: | C, 50.22; | H, 5.45; | N, 17.24. |

### EXAMPLE 24

### Ethyl 3-(4-Ethylamino-1-methanesulfanyl-pyrimidin-5-yl)acrylate

To a room temperature solution of 4-ethylamino-2-methanesulfanyl-pyrimidine-5-carboxaldehyde (6.34 g, 32.14 mmol) in 100 mL of tetrahydrofuran was added (carbethoxymethylene)triphenylphosphorane (14.32 g, 41.14 mmol). The reaction mixture was heated at reflux for 70 minutes then concentrated in vacuo and the residue partitioned between ethyl acetate and 1 N HCl. The organic layer was extracted with additional 1 N HCl, the acidic layers were combined and treated with saturated sodium bicarbonate until basic. The product was extracted into ethyl acetate, and the organic layer was dried over magnesium sulfate, filtered, and concentrated. Upon the addition of hexane, a solid formed. The solid was collected by filtration to give 6.79 g (79%) of ethyl 3-(4-ethylamino-2-methanesulfanyl-pyrimidin-5-yl)acrylate. An analytical sample was obtained by flash chromatography eluting with ethyl acetate, mp 79-80°C.

| Analysis calculated for C₁₂H₁₇N₃O₂S: | | | |
|---|---|---|---|
| | C, 53.91; | H, 6.41; | N, 15.72. |
| Found: | C, 53.97; | H, 6.52; | N, 15.78. |

### EXAMPLE 25

### Ethyl 3-(4-Methytamino-2-methanesulfanyl-pyrimidin-5-yl)acrylate

To a room temperature solution of 4-methylamino-2-methanesulfanyl pyrimidine-5-carboxaldehyde (5.00 g, 27.30 mmol) in 90 mL of tetrahydrofuran was added (carbethoxymethylene)triphenylphosphorane (12.35 g, 35.49 mmol). The reaction mixture was heated at reflux for 2.5 hours then cooled to room temperature and concentrated in vacuo. The residue was partitioned between ethyl acetate and 1 N HCl. The organic layer was treated with saturated sodium bicarbonate until basic. The product was extracted into ethyl acetate and the organic layer dried over magnesium sulfate, filtered, and concentrated. Upon the addition of 4:1 hexane:ethyl acetate, a solid formed that was collected by filtration to give 5.76 g (83%) of ethyl 3-(4 methylamino-2-methanesulfanyl-pyrimidin-5-yl)acrylate, mp 142-144°C.

| Analysis calculated for C₁₁H₁₅N₃O₂S: | | | |
|---|---|---|---|
| | C, 52.16; | H, 5.97; | N, 16.59. |
| Found: | C, 51.89; | H, 5.87; | N, 16.38. |

### EXAMPLE 28

### Ethyl 3-(4-Amino-1-phenylamino-pyrimidin-5-yl)acrylate

To a 0°C solution of 4-amino-2-phenylamino-pyrimidine-5-carbonitrile (7.00 g, 33.18 mmol) (literature prep: J. Org. Chem., 1960:5711) in 170 mL of tetrahydrofuran was added 45 mL of a 1 M solution of diisobutylaluminum hydride in methylene chloride. The ice bath was removed, and an additional 40 mL of a 1 M solution of diisobutylaluminum hydride in methylene chloride was added. The reaction mixture was cooled to 0°C, and 60 mL of methanol was added dropwise. This mixture was then added to a rapidly stirring mixture of 300 mL of ethyl acetate and 250 mL of I N HCl. The layers were separated, and the organic layer was extracted with additional 1 N HCl. The acid layers were combined, treated with 330 mL of 1 N NaOH, and extracted with ethyl acetate. The organic layer was dried over magnesium sulfate, filtered, and concentrated. Purification by flash chromatography eluting with ethyl acetate gave 4.99 g (68%) of 4-amino-2-phenylamino-pyrimidine-5-carboxaldehyde.

To a room temperature solution of 4-amino-2-phenylamino-pyrimidine-5-carboxaldehyde (2.89 g, 13.50 mmol) in 120 mL of tetrahydrofuran was added (carbethoxymethylene)triphenylphosphorane (11.00 g, 31.60 mmol). The reaction mixture was heated at reflux for 9 hours then stirred at room temperature overnight. The solution was concentrated in vacuo and treated with ethyl acetate and hexane to give a yellow solid. The solid was collected by filtration and purified by flash chromatography to give 1.55 g (40%) of ethyl 3-(4-amino-2-phenylamino-pyrimidin-5-yl)acrylate, mp 190-192°C.

| Analysis calculated for C₁₅H₁₆N₄O₂: | | | |
|---|---|---|---|
| | C, 63.37; | H, 5.67; | N, 19.71. |
| Found: | C, 63.08; | H, 5.72; | N, 19.72. |

### EXAMPLE 68

### 3-(4-Ethylamino-2-phenylamino-pyrimidin-5-yl)propionic acid ethyl ester

A mixture of ethyl 3-(4-ethylamino-2-phenylamino-pyrimidin-5-yl)acrylate (152 mg, 0.48 mmol) and 5% palladium on carbon in a solvent mixture of ethanol and tetrahydrofuran was hydrogenated under pressure. The catalyst was filtered off and the filtrate concentrated. The residue was purified by flash chromatography eluting with 2:1 ethyl acetate:hexane to give 72 mg (47%) of 3-(4-ethylamino-2-phenylamino-pyrimidin-5-yl)propionic acid ethyl ester, mp 106-107°C.

| Analysis calculated for C₁₇H₂₂N₄O₂: | | | |
|---|---|---|---|
| | C, 64.95; | H, 7.05; | N, 17.82. |
| Found: | C, 64.90; | H, 7.06; | N, 17.77. |

### EXAMPLE 70

### 3-(4-Methylamino-2-methanesulfanyl-pyrimidin-5-yl)-acrylonitrile

To a room temperature suspension of sodium hydride (240 mg of a 60% suspension of NaH in oil) in 10 mL of dimethylformamide was added diethyl cyanomethylphosphonate (1.0 mL, 6.17 mmol). The reaction mixture was stirred at room temperature for 15 minutes, then 4-methylamino-2-methanesulfanyl-pyrimidine-5-carbaldeyde (1.02 g, 5.57 mmol) in 10 mL of dimethylformamide was added, and the mixture was stirred at room temperature for 10 minutes. The reaction mixture was partitioned between brine and a 1:1 mixture of hexane and ethyl acetate. The organic layer was washed with water, dried over magnesium sulfate, and concentrated to provide 367 mg (32%) of 3-(4-methylamino-2-methanesulfanyl-pyrimidin-5-yl)acrylonitrile, mp 207-210°C. The residue was purified by flash chromatography eluting with 1:1 ethyl acetate:hexane to provide an additional 19 mg (13%) of product.

| Analysis calculated for C₉H₁₀N₄S-0.5 H₂O: | | | |
|---|---|---|---|
| | C, 50.20; | H, 5.15; | N, 26.02. |
| Found: | C, 50.48; | H, 4.80; | N, 26.28. |

### EXAMPLE 72

### 3-(4-Ethylamino-2-phenylamino-pyrimidin-5-yl)acrylonitrile

To a room temperature suspension of sodium hydride (38 mg of a 60% suspension ofNaH in oil) in 5 mL of dimethylformamide was added diethyl cyanomethylphosphonate (150 µL, 0.93 mmol). The reaction mixture was stirred at room temperature for 15 minutes, then 4-ethylamino-2-phenylamino-pyrimidine-5-carbaldeyde (200 mg, 0.83 mmol) in 2 mL of dimethylformamide was added, and the mixture was stirred at room temperature for 10 minutes. The reaction mixture was partitioned between brine and ethyl acetate. The organic layer was washed with water, dried over magnesium sulfate, and concentrated in vacuo The residue was purified by flash chromatography eluting with 1:1 ethyl acetate:hexane. The fractions containing product were concentrated, and hexane was added to the residue. The resultant solid was collected by filtration to give 91 mg (43%) of 3-(4-ethylamino-2-phenylamino-pyrimidin-5-yl)acrylonitrile, mp 244-246°C. Concentration of the filtrate provided an additional 68 mg (32%) of product.

| Analysis calculated for C₁₅H₁₅N₅: | | | |
|---|---|---|---|
| | C, 67.91; | H, 5.70; | N, 26.40. |
| Found: | C, 67.80; | H, 5.57; | N, 26.39. |

### EXAMPLE 73

### 3-(4-Ethylamino-2-phenylamino-pyrimidin-5-yl)-but-2-enoic acid ethyl ester

To a room temperature solution of 4-ethylamino-2-phenylamino-pyrimidine-5-carboxaldehyde (200 mg, 0.83 mmol) in 10 mL of tetrahydrofuran was added (carbethoxyethylidene)triphenylphosphorane (360 mg, 1.0 mmol). The reaction mixture was heated at reflux overnight, cooled, and concentrated in vacuo. The residue was purified by flash chromatography eluting with 1:1 ethyl acetate:hexane. The fractions containing product were concentrated, and 1:2 ethyl acetate:hexane was added to the residue. The resultant solid was collected by filtration to provide 176 mg (65%) of 3-(4-ethylamino-2-phenylamino-pyrimidin-5-yl)-but-2-enoic acid ethyl ester, mp 148-150°C.

| Analysis calculated for C₁₈H₂₂N₄O₂: | | | |
|---|---|---|---|
| | C, 6624; | H, 6.79; | N, 17.16. |
| Found: | C, 65.95; | H, 6.68; | N, 17.02. |

### EXAMPLE 94

### 2-Methanesulfanyl-4-phenylamino-pyrimidine-5-carboxylic acid ethyl ester

To a room temperature solution of 4-chloro-2-methanesulfanyl-pyrimidine-5-carboxylic acid ethyl ester (9.25 g , 40.0 mmol) in 100 mL of tetrahydrofuran was added 16 mL of triethylamine followed by aniline (4.0 mL, 43.8 mmol). The solution was stirred at room temperature overnight. The white solid was removed by filtration washing with ethyl acetate. The filtrate was concentrated in vacuo and partitioned between chloroform and saturated aqueous sodium bicarbonate. The organic layer was dried over magnesium sulfate, filtered, and concentrated. A solution of 2:1 hexane:ethyl acetate was added to the residue, and the resultant white solid was collected to provide 7.07 g (60%) of product. An additional 2.18 g (18%) was obtained from the filtrate. Recrystallization from hexane and ethyl acetate provided an analytical sample of 2-methanesulfanyl-4-phenylamino-pyrimidine-5-carboxylic acid ethyl ester, mp 86-87.5°C.

| Analysis calculated for C₁₄H₁₅N₃O₂S: | | | |
|---|---|---|---|
| | C, 58.11; | H, 5.23; | N, 14.52. |
| Found: | C, 57.93; | H, 5.27; | N, 14.46. |

### EXAMPLE 95

### (2-Methanesulfanyl-4-phenylamino-pyrimidin-5-yl)-methanol

A solution of 2-methanesulfanyl-4-phenylamino-pyrimidine-5-carboxylic acid ethyl ester (7.25 g, 25.1 mmol) in 100 mL of tetrahydrofuran was added dropwise to a room temperature suspension of lithium aluminum hydride (1.55 g, 40.9 mmol) in 100 mL of tetrahydrofuran. After 10 minutes, an additional 1.00 g of lithium aluminum hydride was added to the reaction mixture, and stirring was continued for 1.5 hours. The reaction was carefully quenched with isopropanol followed by 6 mL of water, 10 mL of 15% NaOH, and 20 mL of water, and the mixture was stirred for 1.5 hours. The white precipitate was removed by filtration washing with ethyl acetate. The filtrate was washed with water, dried over magnesium sulfate, filtered, and concentrated in vacuo. Purification by flash chromatography eluting with ethyl acetate provided 2.22 g (36%) of (4 ethylamino-2-methanesulfanyl-pyrimidin-5-yl)-methanol, mp 127-128°C.

| Analysis calculated for C₁₂H₁₃N₃OS: | | | |
|---|---|---|---|
| | C, 58.28; | H, 5.30; | N, 16.99. |
| Found: | C, 58.15; | H, 5.09; | N, 16.90. |

### EXAMPLE 96

### 2-Methanesulfanyl-4-phenylamino-pyrimidine-5-carboxaldehyde

To (4-ethylamino-2-methanesulfanyl-pyrimidin-5-yl)-methanol (2.80 g, 11.4 mmol) in 400 mL of chloroform was added manganese oxide (3.95 g, 45.4 mmol). The suspension was stirred at room temperature overnight. The mixture was filtered through celite washing with chloroform. The filtrate was concentrated in vacuo to give 2.73 g (98%) of 2-methanesulfanyl-4-phenylamino-pyrimidine-3-carboxaldehyde, mp 89-90°C.

| Analysis calculated for C₁₂H₁₁N₃OS: | | | |
|---|---|---|---|
| | C, 58.76; | H, 4.52; | N, 17.13. |
| Found: | C, 58.56; | H, 4.69; | N, 17.10. |

### EXAMPLE 97

### Ethyl 3-(2-Methanesulfanyl-4-phenylamino-pyrimidin-5-yl)acrylate

To a room temperature solution of 2-methanesulfanyl-4-phenylamino pyrimidine-5-carboxaldehyde (1.00 g, 4.08 mmol) in 20 mL of tetrahydrofuran was added (carbetboxymethylene)triphenylphosphorane (1.82 g, 5.22 mmol). The reaction mixture was heated at reflux for 70 minutes, then concentrated in vacuo and partitioned between ethyl acetate and 1N hydrochloric acid. The organic layer was extracted with two additional portions of 1N hydrochloric acid, and the acid layers were combined and neutralized with saturated sodium bicarbonate. The product was extracted into ethyl acetate, dried over magnesium sulfate, filtered, and concentrated in vacuo. The residue was purified by flash chromatography eluting with ethyl acetate to provide 988 mg (77%) of ethyl 3-(2-methanesulfanyl-4-phenylamino-pyrimidin-5-yl)acrylate as a yellow oil.

### EXAMPLE 102

### 4-Cyclopentylamino-2-methanesulfanyl-pyrimidine-5-carboxylic acid ethyl ester

To a room temperature solution of 4-chloro-2-methanesulfanyl-pyrimidine-5-carboxylic acid ethyl ester (12.48 g, 53.8 mmol) in 150 mL of tetrahydrofuran was added 22 mL of triethylamine followed by cyclopentylamine (6.70 g, 77.0 mmol). The solution was stirred at room temperature for 1 hour. The white solid was removed by filtration washing with ethyl acetate. The filtrate was concentrated in vacuo and partitioned between ethyl acetate and saturated aqueous sodium bicarbonate. The organic layer was washed with brine, dried over magnesium sulfate, filtered, and concentrated. A solution of 2:1 hexane:ethyl acetate was added to the residue, and the resultant white solid was collected to provide 13.3 g (88%) of 4-cyclopentylamino-2-methanesulfanyl-pyrimidine-5-carboxylic acid ethyl ester as an oil.

| Analysis calculated for C₁₃H₁₉N₃O₂S: | | | |
|---|---|---|---|
| | C, 55.49; | H, 6.81; | N, 14.93. |
| Found: | C, 55.59; | H, 6.72; | N, 14.85. |

### EXAMPLE 103

### (4-Cyclopentylamino-2-methanesulfanyl-pyrimidin-5-yl)-methanol

A solution of 4-cyclopentylamino-2-methanesulfanyl-pyrimidine-5-carboxylic acid ethyl ester (13.0 g, 46.3 mmol) in 50 mL of tetrahydrofuran was added dropwise to a room temperature suspension of lithium aluminum hydride (3.2 g, 84.2 mmol) in 150 mL of tetrahydrofuran. The reaction mixture was stirred at room temperature for 20 minutes, then carefully quenched with 6 mL of water, followed by 6 mL of 15% NaOH and 19 mL of water. After stirring for 1 hour, the white precipitate was removed by filtration washing with ethyl acetate. The filtrate was concentrated in vacuo, and hexane and ethyl acetate were added to the residue. Filtration of the white solid provided 8.39 g (76%) of (4-cyclopentylamino-2-methanesulfanyl-pyrimidin-5-yl)-methanol, mp 127-128°C.

| Analysis calculated for C₁₁H₁₇N₃OS-0.1 H₂O: | | | |
|---|---|---|---|
| | C, 54.79; | H, 7.19; | N, 17.43. |
| Found: | C, 54.68; | H, 7.12; | N, 17.23. |

### EXAMPLE 104

### 4-Cyclopentylamino-2-methanesulfanyl-pyrimidine-5-carboxaldehyde

To (4-cyclopentylamino-2-methanesulfanyl-pyrimidin-5-yl)-methanol (8.00 g, 33.5 mmol) in 400 mL of chloroform was added manganese oxide (18.5 g, 213 mmol). The suspension was stirred at room temperature overnight. An additional amount of manganese oxide (2.5 g, 29 mmol) was added, and stirring was continued for 2.5 h. The mixture was filtered through celite washing with chloroform. The filtrate was concentrated in vacuo to give 7.93 g (99%) of 4-cyclopentylamino-2-methanesulfanyl-pyrimidine-5-carboxaldehyde as an oil.

| Analysis calculated for C₁₁H₁₅N₃OS: | | | |
|---|---|---|---|
| | C, 55.67; | H, 6.37; | N, 17.71. |
| Found: | C, 55.60; | H, 6.24; | N, 17.70. |

### EXAMPLE 105

### Ethyl 3-(4-Cyclopentylamino-2-methanesulfanyl-pyrimidin-5-yl)acrylate

To a room temperature solution of 4-cyclopentylamino-2-methanesulfanyl-pyrimidine-5-carboxaldehyde (7.74 g, 32.7 mmol) in 110 mL of tetrahydrofuran was added (carbethoxymethylene)triphetrylphosphorane (15.0 g, 43.1 mmol). The reaction mixture was heated at reflux for 1.5 hours, then cooled to room temperature and partitioned between ethyl acetate and 1N hydrochloric acid. Concentrated aqueous sodium hydroxide was added to the acid layer followed by extraction of the product into ethyl acetate. The organic layer was dried over magnesium sulfate, filtered, and concentrated in vacuo. The residue was purified by flash chromatography eluting with 4:1 hexane:ethyl acetate to provide 6.58 g (66%) of ethyl 3-(4-cyclopentylamino-2-methanesulfanyl-pyrimidin-5-yl)acrylate, mp 98-101 °C.

| Analysis calculated for C₁₅H₂₁N₃O₂S: | | | |
|---|---|---|---|
| | C, 58.61; | H, 6.89; | N, 13.67. |
| Found: | C, 58.57; | H, 6.83; | N, 13.52. |

### EXAMPLES 109-271

The following invention compounds were similarly prepared by following the general procedures of the foregoing examples.

### EXAMPLE 119

### 4-Cyclohexylamino-2-methylsulfanyl-pyrimidine-5-carboxylic acid ethyl ester, oil.

### EXAMPLE 120

### 4-Cyclopropylamino-2-methylsulfanyl-pyrimidine-5-carboxylic acid ethyl ester, oil.

### EXAMPLE 121

### (4-Cyclohexylamino-2-methylsulfanyl-pyrimidin-5-yl)-methanol, mp 127-129°C.

### EXAMPLE 122

### 4-Cyclohexylamino-2-methylsulfanyl-pyrimidine-5-carboxaldehyde, oil.

### EXAMPLE 123

### 3-(4-Cyclohexylamino-2-methysulfanyl-pyrimidin-5-yl)-acrylic acid ethyl ester

### EXAMPLE 124

### (4-Cyclopropylamino-2-methylsulfanyl-pyrimidin-5-yl)-methanol, mp 134-135°C.

### EXAMPLE 125

### 4-Cyclopropylamino-2-methylsulfanyl-pyrimdine-5-carboxaldehyde, mp 63-64°C.

### EXAMPLE 128

### 3-(4-Cyclopropylamino-2-methylsulfanyl-pyrimidin-5-yl)-acrylic acid ethyl ester, oil.

### EXAMPLE 149

### (4-Cycloheptylamino-2-methylsulfanyl-pyrimidin-5-yl)-methanol, mp 141-143°C.

### EXAMPLE 168

### 1-(4-Nitro-phenyl)-pyrrolidine-2-carboxylic acid tert-butyl ester (S), mp 103-104°C.

### EXAMPLE 169

### 1-(4-Amino-phenyl)-pyrrolidine-2-carboxylic add tert-butyl ester (S), mp 75-76°C.

### EXAMPLE 172

### [1-(4-Nitro-phenyl)-piperidin-3-yl]-methanol (racemic), mp 99-100°C.

### EXAMPLE 173

### [1-(4-Amino-phenyl)-piperidin-3-yl]-methanol (racemic), mp 108-110°C.

### EXAMPLE 174

### [4-(Bicyclo[2.2.1]hept-2-ylamino)-2-methylsulfanyl-pyrimidin-5-yl]-methanol (exo), mp 117-118°C.

### EXAMPLE 186

### 2-[1-(4-Nitro-phenyl)-piperidin-4-yl]-ethanol, mp 60-61°C.

### EXAMPLE 187

### 3-[1-(4-Nitro-phenyl)-piperidin-4-yl]-propan-1-ol, mp 166-167°C.

### EXAMPLE 188

### 2-[1-(4-Amino-phenyl)-piperidin-4-yl]-ethanol, mp 121-122°C.

### EXAMPLE 189

### 3-[1-(4-Amino-phenyl)-piperidin-4-yl]-propan-1-ol, mp 98-99°C.

### EXAMPLE 192

### [1-(4-Nitro-phenyl)-piperidin-2-yl]-methanol, mp 68-69°C.

### EXAMPLE 193

### 1-(4-Nitro-phenyl)-piperidin-4-ol, mp 99-100°C.

### EXAMPLE 194

### 1-(4-Amino-phenyl)-piperidin-4-ol, mp 168-169°C.

### EXAMPLE 199

### [1-(4-Amino-phenyl)-piperidin-2-yl]-methanol, mp 91-92°C.

### EXAMPLE 204

### 1-(4-Nitro-phenyl)-piperidin-3-ol, mp 112-113C.

### EXAMPLE 205

### 1-(4-Amino-phenyl)-piperidin-3-ol, mp 101-102C.

### EXAMPLE 208

### Dimethyl-[1-(4-nitro-phenyl)-piperidin-4-yl]-amine, mp 102-103°C.

### EXAMPLE 209

### 1'-(4-Nitro-phenyl)-[1,4']bipiperidinyl, mp 90-91°C.

### EXAMPLE 210

### [1-(4-Amino-phenyl)-piperidin-4-yl]-dimethyl-amine, mp 126-127°C.

### EXAMPLE 222

### 2-Benzylamino-8-cyclohexyl-8H-pyrido[2,3-d]pyrimidin-7-one, mp 183-184°C.

### EXAMPLE 238

### 3-{4-[2-(tert-Butyl-dimethyl-silanyloxy)-cyclopentylamino]-2-methylsulfanyl-pyrimidin-5-yl}-acrylic acid ethyl ester,

MS (CI) m/z 438 (M⁺).

### EXAMPLE 243

### 4-[5-(2-Ethoxycarbonyl-vinyl)-2-methylsulfanyl-pyrimidin 4 yl amino]-piperidine-1-carborylic acid ethyl ester, oil.

MS (CI) m/z 395 (M + 1).

### EXAMPLE 244

### 4-(2-Methanesulfanyl-7-oxo-7H-pyrido[2,3-d]pyrimidin-8-yl)piperidine-1-carboxylic acid ethyl ester, mp 165-167C.

### EXAMPLE 245

### 4-(2-Methanesulfinyl-7-oxo-7H-pyrido[2,3-d]pyrimidin-8-yl)-piperidine-1-carboxylic acid ethyl ester, mp 151-154°C.

MS (CI) m/z 365 (M + 1).

### EXAMPLE 246

### 4-[7-oxo-2-(4-piperidin-1-yl-phenylamino)-7H-pyrido[2,3-d]pyrimidin-8-yl]-piperidine-1-carboxylic acid ethyl ester, mp 231-233°C.

### EXAMPLE 248

### 2-(3-Bromo-2,2-dimethyl-propoxy)-tetrahydro-pyran, oil.

As noted above, the compounds of this invention are potent inhibitors of cyclin-dependent kinases, and accordingly, are useful in treating and preventing atherosclerosis, and other cell proliferative disorders like cancer. The compounds have exhibited excellent inhibitory activity against a wide variety of cyclin-dependent kinases, all in assay systems routinely utilized to measure such activity. A typical assay, for instance, measures inhibitory activity against the cyclin D dependent kinase 4 enzyme (cdk4/D). The invention compounds of Formulas I and II exhibited IC₅₀ values ranging generally from 0.0045 µM to 10 µM. The cdk 4 assay was carried out as follows.

### Cyclin-Dependent Kinase 4 (cdk4) Assay

Enzyme assays for IC₅₀ determinations (Tables 1 and 2) and kinetic evaluation were performed in 96 well filter plates (Millipore MADVN6550). The total volume was 0.1 mL containing a final concentration of 20 mM TRIS (tris[hydroxymethyl]aminomethane), at pH 7.4, 50 mM NaCl, 1 mM dithiothreitol, 10 mM MgCl₂, 25 µM ATP containing 0.25 µCi of [³²P]ATP, 20 ng of cdk4, 1 µg of retinoblastoma, and appropriate dilutions of a compound of the present invention. All components except the ATP were added to the wells, and the plate was placed on a plate mixer for 2 minutes. The reaction was started by adding [³²P]ATP and the plate was incubated at 25°C for 15 minutes. The reaction was terminated by addition of 0.1 mL of 20% trichloroacetic acid (TCA). The plate was kept at 4°C for at least 1 hour to allow the substrate to precipitate. The wells were then washed five times with 0.2 mL of 10% TCA and ³²P incorporation was determined with a beta plate counter (Wallac Inc., Gaithersburg, MD).

### Cyclin-Dependent Kinase Assays (cdk2/cyclinE, cdk2/cyclinA, cdc2/cyclinB)

Enzyme assays for IC₅₀ determinations and kinetic evaluation were performed in a 96-well filter plate (Millipore MADVN6550) in a total volume of 0.1 mL of 20 mM TRIS (tris[hydroxymethyl]aminomethane), at pH 7.4, 50 mM NaCl, 1 mM dithiothreitol, 10 mM MgCl₂, 12 mM ATP containing 0.25 µCi of [³²P]ATP, 20 ng of enzyme (either cdk2/cyclinE, cdk2/A, or cdc2/cyclinB), 1 µg retinoblastoma, and appropriate dilutions of the particular invention compound. All components except the ATP were added to the wells, and the plate was placed on a plate mixer for 2 minutes. The reaction was begun by addition of [³²P]ATP, and the plate was incubated at 25°C for 15 minutes. The reaction was terminated by addition of 0.1 mL of 20% TCA. The plate was kept at 4°C for at least 1 hour to allow the substrate to precipitate. The wells were then washed five times with 0.2 mL of 10% TCA and ³²P incorporation determined with a beta plate counter (Wallac Inc., Gaithersburg, MD).

When measured against cdk2/E, the invention compounds exhibited IC₅₀ values ranging generally from about 0.02 to about 25 µM. Against cdk2/A, the compounds exhibited IC₅₀ values ranging from about 0.01 to about 14 µM, and against cdk2/B, generally from about 0.06 to about 40 µM. The assays were carried out as described above, and specific data is given in Table 2.

**TABLE2**

| | | | | | | | |
|---|---|---|---|---|---|---|---|
| 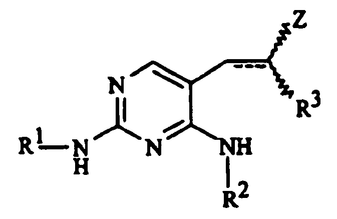 | | | | | | | |

| Example | R₁ | R₂ | R₃ | Bond | Z | cdk4/IC₅₀ IC₅₀ µM | cdk4/D % inhibition at 40 µM |
|---|---|---|---|---|---|---|---|
| 17 | Ph | Et | H | trans double | COOEt | 2 | |
| 68 | Ph | Et | H | single | COOEt | 90 | 37% |
| 28 | Ph | H | H | trans double | COOEt | 65 | |
| 73 | Ph | Et | Me | trans double | COOEt | | 58% |
| 72 | Ph | Et | H | trans double | CN | | 18% |

Several of the invention compounds have also shown good inhibitory activity against cdk6/D₂ and cdk6/D₃ enzymes. These assays are carried out in a manner similar to that described above for cdk4, by simply employing the appropriate cdk6 kinase enzyme. Invention compounds have shown IC₅₀ values ranging from about 0.009 µM to about 0.2 µM. The compound of Example 214, for instance, had an IC₅₀ of 0.0071 µM against cdk6/D₂, and an IC₅₀ of 0.013 µM against cdk6/D₃.

The compounds of Formulas II also have shown good inhibitory activity against certain growth factor receptor tyrosine kinase enzymes, including fibroblast growth factor (FGF) and platelet derived growth factor (PDGF). The compounds exhibit only marginal activity against epidermal growth factor (EGF) receptor tyrosine kinase. The invention compounds range in IC₅₀ inhibition against FGF tyrosine kinase generally from about 0.004 to about 40 µM. Against PDGF tyrosine kinase, the invention compounds exhibit IC₅₀ from about 0.05 to about 40 µM. The assays used to determine these activities were carried out as follows:

### Purification of Epidermal Growth Factor Receptor Tyrosine Kinase

Human EGF receptor tyrosine kinase was isolated from A431 epidermoid carcinoma cells by the following method. Cells were grown in roller bottles in 50% Dulbecco's Modified Eagle medium and 50% HAM F-12 nutrient media (Gibco) containing 10% fetal calf serum. Approximately 10⁹ cells were lysed in two volumes of buffer containing 20 mM 2-(4N-[2-hydroxymethyl]-piperazin-1-yl)ethanesulfonic acid, pH 7.4, 5 mM ethylene glycol bis(2-aminoethyl ether) N,N,N',N'-tetraacetic acid, 1% Triton X-100, 10% glycerol, 0.1 mM sodium orthovanadate, 5 mM sodium fluoride, 4 mM pyrophosphate, 4 mM benzamide, 1 mM dithiothrietol, 80 µg/mL aprotinin, 40 µg/mL leupeptin, and 1 mM phenylmethylsulfonylfluoride (PMSF). After centrifugation at 25,000 x g for 10 minutes, the supernatant was equilibrated for 2 hours at 4µC with 10 mL of wheat germ agglutinin sepharose that was previously equilibrated with 50 mM Hepes, 10% glycerol, 0.1% Triton x-100 and 150 mM NaCl, pH 7.5 (equilibration buffer). Contaminating proteins were washed from the resin with 1 M NaCl in equilibration buffer, and the enzyme was eluted with 0.5 M N-acetyl-1-D-glucosamine in equilibration buffer.

### PDGF and EGF Receptor Tyrosine Kinase Assays

Full-length cDNAs for the mouse PDGF-β and human FGF-1 (flg) receptor tyrosine kinases were obtained from J. Escobedo and prepared as described in J. Biol. Chem., 1991;262:1482-1487. PCR primers were designed to amplify a fragment of DNA that codes for the intracellular tyrosine kinase domain. The fragment was inserted into a baculovirus vector, cotransfected with AcMNPV DNA, and the recombinant virus isolated. SF9 insect cells were infected with the virus to overexpress the protein, and the cell lysate was used for the assay. Assays were performed in 96-well plates (100 µL/incubation/well), and conditions were optimized to measure the incorporation of ³²P from γ³²P-ATP into a glutamate-tyrosine co-polymer substrate. Briefly, to each well was added 82.5 µL of incubation buffer containing 25 mM Hepes (pH 7.0), 150 mM NaCl, 0.1% Triton X-100, 0.2 mM PMSF, 0.2 mM Na₃VO₄, 10 mM MnCl₂, and 750 µg/mL of Poly (4:1) glutamate-tyrosine followed by 2.5 µL of inhibitor and 5 µL of enzyme lysate (7.5 µg/µL FGF-TK or 6.0 µg/µL PDGF-TK) to initiate the reaction. Following a 10 minute incubation at 25°C, 10 mL of γ³²P-ATP (0.4 µCi plus 50 µM ATP) was added to each well, and samples were incubated for an additional 10 minutes at 25°C. The reaction was terminated by the addition of 100 µL of 30% trichloroacetic acid (TCA) containing 20 mM sodium pyrophosphate and precipitation of material onto glass fiber mats (Wallac). Filters were washed three times with 15% TCA containing 100 mM sodium pyrophosphate, and the radioactivity retained on the filters counted in a Wallac 1250 Betaplate reader. Nonspecific activity was defined as radioactivity retained on the filters following incubation of samples with buffer alone (no enzyme). Specific enzymatic activity (enzyme plus buffer) was defined as total activity minus nonspecific activity. The concentration of a compound that inhibited specific activity by 50% (IC₅₀) was determined based on the inhibition curve, and typical results are reported in Table 4.

**TABLE4**

| | | | | | | | |
|---|---|---|---|---|---|---|---|
| 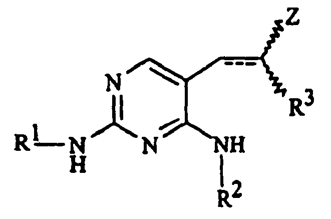 | | | | | | | |

| Example | R¹ | R² | R³ | Z | Bond | PDGF | FGF IC₅₀ µM |
|---|---|---|---|---|---|---|---|
| 17 | Ph | Et | H | COOEt | trans double | 3.7 | 4.5 |

The Src family of protein kinases, which all contain a SH2 domain, are involved in a number of cellular signaling pathways. For example, Src is involved in growth factor receptor signaling; integrin-mediated signaling; T- and B-cell activation and osteoclast activation. It is known that the Src SH2 domain binds to several key receptor and nonreceptor tyrosine kinases such as tyrosine kinases containing receptors for PDGF, EGF, HER2/Neu (an oncogene form of EGF), FGF, focal adhesion kinase, p130 protein, and p68 protein. In addition, pp60c-Src has been shown to be involved in the regulation of DNA synthesis, mitosis, and other cellular activities.

Thus, it would be useful to have compounds that inhibit the binding of proteins containing an SH2 domain to cognate phosphorylated proteins, as the inhibition of binding of proteins containing an SH2 domain to cognate phosphorylated proteins can be used to treat proliferative diseases such as cancer, osteoporosis, inflammation, allergy, restenosis, and cardiovascular disease, which all rely on signal transduction involving proteins that contain an SH2 domain that binds to phosphorylated proteins during the cellular signaling process.

Several of the invention compounds have been evaluated in a standard assay to measure their ability to inhibit cellular Src protein kinase (c-Src). The invention compounds exhibited IC₅₀ values ranging generally from about 0.1 to about 50 µM. The assay was carried out as follows:

C-Src kinase was purified from baculovirus infected insect cell lysates using an antipeptide monoclonal antibody directed against the N-terminal amino acids (amino acids 2-17) of c-Src. The antibody, covalently linked to 0.65 µm latex beads, was added to a suspension of insect cell lysis buffer comprised of 150 mM NaCl, 50 mM Tris pH 7.5, 1 mM DTT, 1% NP-40, 2 mM EGTA, 1 mM sodium vanadate, 1 mM PMSF, 1 µg/mL each of leupeptin, pepstatin, and aprotinin. Insect cell lysate containing c-Src protein was incubated with these beads for 3 to 4 hours at 4°C with rotation. At the end of the lysate incubation, the beads were rinsed three times in lysis buffer, resuspended in lysis buffer containing 10% glycerol, and frozen. These latex beads were thawed, rinsed three times in assay buffer (40 mM Tris, pH 7.5, 5 mM µgCl₂) and suspended in the same buffer. In a Millipore 96-well plate with a 0.65 µm polyvinylidine membrane bottom were added the reaction components: 10 µL c-Src beads, 10 µL of 2.5 mg/mL poly GluTyr substrate, 5 µM ATP containing 0.2 µCi labeled ³²P-ATP, 5 µL DMSO containing inhibitors or as a solvent control, and buffer to make the final volume 125 µL. The reaction was started at room temperature by addition of the ATP and quenched 10 minutes later by the addition of 125 µL of 30% TCA, 0.1 M sodium pyrophosphate for 5 minutes on ice. The plate was then filtered and the wells washed with two 250 mL aliquots of 15% TCA, 0.1 M pyrophosphate. The filters were then punched, counted in a liquid scintillation counter, and the data examined for inhibitory activity in comparison to a known inhibitor such as erbstatin. The method is also described in J. Med. Chem., 1994;37:598-609.

The invention compounds additionally have been shown to be bioavailable in animals, reaching peak plasma levels in nude mice in the range of about 10 nM to about 200 nM within 30 minutes following oral dosing at levels of about 4 to 5 mg/kg as suspensions in lactate buffer solutions having pH of 4.0. For example, the compound of Example 60 was administered orally at 5 mg/kg to mice, and plasma levels of about 200 nM were measured at 30 minutes following dosing. The compound was also administered intraperitoneally at 12 mg/kg and produced a peak plasma concentration of 10,000 nM at 30 minutes following dosing. When evaluated in female nude mice bearing subcutaneous MCF-7 human mammary tumor xenografts, the compound of Example 60 showed statistically insignificant tumor growth inhibitions at doses of 5 to 20 mg/kg when dosed on a schedule of q12h × 2; days 1-14.

The invention compounds can be formulated in conventional manners to provide convenient dosage forms for delivery to mammals by various routes, including oral, parenteral (i.e., subcutaneous, intravenous, and intramuscular), transdermal, e.g. slow release skin patch or cream, as well as by slow release delivery devices such as osmotic pumps, suppositories, and buccal seals. The following examples further illustrate how the compounds are readily formulated.

### EXAMPLE 272

### 50 mg Tablet Formulation

| Per Tablet | | Per 10,000 Tablets |
|---|---|---|
| 0.050 g | 2-benzylamino-8-cyclopropyl-8H-pyrido[2,3-d]pyrimidin-7-one | 500 g |
| 0.080 g | lactose | 800 g |
| 0.010 g | corn starch (for mix) | 100 g |
| 0.008 g | corn starch (for paste) | 80 g |
| 0.148 g | | 1480 g |
| 0.002 g | magnesium stearate (1%) | 20 g |
| 0.150 g | | 1500 g |

The pyrido pyrimidine, lactose, and corn starch (for mix) are blended to uniformity. The corn starch (for paste) is suspended in 600 mL of water and heated with stirring to form a paste. This paste is used to granulate the mixed powders. The wet granules are passed through a No. 8 hand screen and dried at 80°C. The dry granules are then passed through a No. 16 screen. The mixture is lubricated with 1% magnesium stearate and compressed into tablets in a conventional tableting machine. The tablets are useful for treating cancers such as breast, prostate, lung, ovarian, colon, pancreatic, melanoma, esophageal, brain, Kaposi's sarcoma, and lymphomas.

### EXAMPLE 273

### Preparation of Oral Suspension

| Ingredient | Amount |
|---|---|
| 8-Ethyl-2-(4-pyrrol-1-yl-phenylamino)-8H-pyrido[2,3-d]pyrimidin-7-one | 500 mg |
| Sorbitol solution (70% N.F.) | 40 mL |
| Sodium benzoate | 150 mg |
| Saccharin | 10 mg |
| Cherry flavor | 50 mg |
| Distilled water qs | 100 mL |

The sorbitol solution is added to 40 mL of distilled water, and the pyrido pyrimidine is suspended therein. The saccharin, sodium benzoate, and flavoring are added and dissolved. The volume is adjusted to 100 mL with distilled water. Each milliliter of syrup contains 5 mg of invention compound.

### EXAMPLE 274

### Preparation of Parenteral Solution

In a solution of 700 mL of propylene glycol and 200 mL of water for injection is suspended 20.0 g of 8-bicyclo[2.2.1]hept-2-yl-2-phenylamino-8H-pyrido[2,3-d]pyrimidin-7-one with stirring. After suspension is complete, the pH is adjusted to 5.5 with hydrochloric acid, and the volume is made up to 1000 mL with water for injection. The formulation is sterilized, filled into 5.0 mL ampoules, each containing 2.0 mL (representing 40 mg of invention compound) and sealed under nitrogen.

### EXAMPLE 275

### Suppositories

A mixture of 400 mg of 8-(2-hydroxy-cyclopentyl)-2-(4-piperidin-1-yl-phenylamino)-8H-pyrido[2,3-d]pyrimidin-7-one, and 600 mg of theobroma oil is stirred at 60°C to uniformity. The mixture is cooled and allowed to harden in a tapered mold to provide a 1 g suppository.

### EXAMPLE 276

### Slow Release Formulation

Five hundred milligrams of 8-(3-hydroxypropyl)-2-(4-piperidin-1-yl-phenylamino)-8H-pyrido[2,3-d]pyrimidin-7-one is converted to a hydrochloride salt and placed into an Oros osmotic pump for controlled release for treatment of atherosclerosis.

### EXAMPLE 277

### Skin Patch Formulation

Fifty milligrams of (8-Bicyclo[2.2.1]hept-2-yl-2-[4-[4-(2-morpholin-4-yl-ethyl)-piperidin-1-yl]-phenylamino]-8H-pyrido[2,3-d]pyrimidin-7-one (exo)) is admixed with 50 mg of propylene glycol monolaurate in a polydimethylsiloxane adhesive. The mixture is layered onto an elastic film made with an adhesive formulation of polybutene, polyisobutylenc, and propylene glycol monolaurate. The layers are placed between 2 layers of polyurethane film. A release liner is attached to the adhesive surface, and is removed prior to application to a skin surface. The propylene glycol monolaurate serves as a permeation-enhancing agent.

## Claims

1. A compound having the general Formula II: 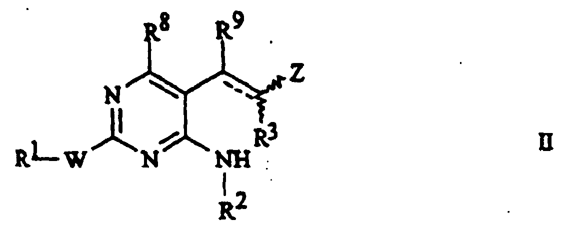 wherein:
the dotted line represents an optional double bond of either trans or cis-stereochemistry;
W is NH, S, SO, or SO2;
Z is COOR⁷, CN, CHO, CH₂0R⁷, CH₂NHR⁷, CONHR⁷, or COR⁷;
R 1 and R 2 are independently selected from the group consisting of
H,
(CH₂)ₙAr wherein aryl is selected from phenyl or naphthyl
(CH₂)ₙheteroaryl, wherein the heteroaryl group have from 4 to 9 ring atoms, from 1 to 4 of which are independently selected from the group consisting of O, S, and N, (CH₂)ₙheterocycle, which means a cycloalkyl group as defined below having at least one hetero atom selected from 0, S, NR₂,
C₁-C₁₀ alkyl,
C₃-C₁₀ cycloalkyl,
C₂-C₁₀ alkenyl, and
C₂-C₁₀ alkynyl,
wherein n is 0, 1, 2, or 3 and the
(CH₂)ₙAr, alkyl, cycloalkyl, alkenyl, and alkynyl groups are optionally substituted by groups of
NR⁴R⁵,
N(O)R⁴R⁵,
NR⁴R⁵R⁶Y,
phenyl,
phenyl substituted by 1, 2, or 3 groups independently selected from the group consisting of C₁-C₁₀ alkyl, C₁-C₁₀ alkoxy, thio, thio C₁-C₁₀ alkyl, hydroxy, -COOR⁷, amino of the formula -NR⁴R⁵, and T(CH₂)ₘQR⁴ or T(CH₂)ₘC0₂R⁴ wherein m is 1 to 6, T is O, S, NR⁴, N(O)R⁴, NR⁴R⁶Y, or CR⁴R⁵, Q is O, S, NR⁵, N(O)R⁵, or NR⁵R⁶Y,
hydroxy,
C₁-C₁₀ alkoxy,
Phenoxy,
thiol,
thioC₁-C₁₀alkyl
halo,
COR⁴,
C0₂R⁴,
CONR⁴R⁵,
SO₂NR⁴R⁵,
SO₃R⁴,
P0₃R⁴,
aldehyde,
nitrile,
nitro,
heteroaryloxy wherein heteroaryl is as defined above,
T(CH₂)ₘQR⁴, C(0)T(CH₂)ₘQR⁴, NHC(O)T(CH₂)ₘQR⁴, or T(CH₂)ₘCO₂R⁴ wherein m is 1 to 6, T is 0, S, NR⁴, N(0)R⁴, NR⁴R⁵Y, or CR⁴R⁵, Q is 0, S, NR⁵, N(0)R⁵,
or NR⁵R⁶Y
R³ is H or C₁-C₁₀alkyl
R⁴ and R⁵ are Independently selected from the group consisting of
hydrogen,
C₁-C₆ alkyl
substituted alkyl as defined above
C₂-C₆ alkenyl,
C₂-C₆ alkynyl,
(CH₂)ₙAr as defined above
C₃-C₁₀ cycloalkyl, or
R⁴ and R⁵ taken together with the nitrogen to which they are attached optionally form a ring having 3 to 7 carbon atoms and said ring optionally contains 1, 2, or 3 heteroatoms selected from the group consisting of
nitrogen,
substituted nitrogen wherein "substituted nitrogen" means nitrogen bearing C₁-C₆ alkyl or (CH₂)ₙPh where n is 1, 2, or 3,
oxygen, and
sulfur;
R⁶ is C₁-C₁₀ alkyl;
Y is a halo counter-ion;
R⁷ is one of H, C₁ -C₁₀ alkyl, or phenyl;
R⁸ and R⁹ independently are
H,
C₁-C₃alkyl,
NR⁴R⁵,
N(O)R⁴R⁵,
NR⁴R⁵R⁶Y,
hydroxy,
alkoxy as defined above,
thiol,
thioalkyl as defined above,
halo,
COR⁴, CO²R⁴,
CONR⁴R⁵, SO₂NR⁴R⁵, SO₃R⁴, PO₃R⁴, CHO, CN, or NO₂;
and the pharmaceutically acceptable salts thereof.

2. A compound of Claim 1 wherein W is NH, and R 8 and R 9 both are hydrogen.

3. A compound of Claim 2 wherein R 1 is phenyl or substituted phenyl as defined above.

4. A compound according to Claim 3 which is:
Ethyl 3-(4-Ethylamino-2-phenylamino-pyrimidin-5-yl)acrylate;
Ethyl 3-(4-Amino-2-phenylamino-pyrimidin-5-yl)acrylate;
3-(4-Ethylamino-2-phenylamino-pyrimidin-5-yl )propionic acid ethyl ester;
3-(4- Ethylamino-2-phenylamino-pyrimidin- 5-yl )acrylonitrile; and
3-(4-Ethylamino-2-phenylamino-pyrimidin-5-yl)-but-2-enoic acid ethyl ester.

5. A compound of Claim 1 wherein W is S, SO, or SO₂.

6. A.compound according to Claim 5 which is:
Ethyl 3-(4-Amino-2-methanesulfanyl-pyrimidin-5-yl)acrylate;
Ethyl 3-(4-Ethylamino-2-methanesulfanyl-pyrimidin-5-yl)acrylate;
Ethyl 3-(4-Methylamino-2-methanesulfanyl-pyrimidin-5-yl)acrylate;
3-(4-Methylamino-2-methanesulfanyl-pyrimidin-5-yl)-acrylonitrile;
Ethyl 3-(4-Cyclopentylamino-2-methanesulfanyl-pyrimidin-5-yl)acrylate;
3-(4-Cyclohexylamino-2-methysulfanyl- pyrimidin-5-yl)-acrylic acid ethyl ester;
3-(4-Cyclopropylamino-2-methylsulfanyl-pyrimidin-5-yl)-acrylic acid ethyl ester; and
4-[5-(2-Ethoxycarbonyl-vinyl)-2-methylsulfanyl-pyrimidin 4 yl anino]-piperidine-1-carboxylic acid ethyl ester.

7. A pharmaceutical formulation comprising a compound selected from Claims 1-6 in combination with a pharmaceutically acceptable carrier, diluent, or excipient.

8. Use of a compound selected from one of Claims 1 - 6 for the manufacture of a pharmaceutical composition for controlling proliferative disorders selected from the group consisting of cancer, psoriasis, vascular smooth muscle proliferation associated with a disorder selected from the group consisting of atherosclerosis, postsurgical vascular stenois, and restenosis in mammals.

9. Use of a compound selected from one of Claims 1 -6 for the manufacture of a pharmaceutical composition for inhibiting a cyclin-dependent kinase.

10. Use of Claim 9 wherein said cyclin-dependent kinase is cdc2.

11. Use of Claim 9 wherein said cyclin-dependent kinase is cdk2.

12. Use of Claim 9 wherein said cyclin-dependent kinase is cdk4 or cdk6.

13. Use of a compound selected from one of Claims 1 - 6 for the manufacture of a pharmaceutical composition for inhibiting a growth factor-mediated tyrosine kinase.

14. Use of Claim 13 wherein said growth factor-mediated tyrosine kinase is platelet derived growth factor (PDGF).

15. Use of Claim 13 wherein said growth factor-mediated tyrosine kinase is fibroblast growth factor (FGF).

16. Use of a compound selected from one of Claims 1 - 6 for the manufacture of a pharmaceutical composition for treating a subject suffering from diseases caused by vascular smooth muscle cell proliferation.

17. Use of a compound selected from one of Claims 1 - 6 for the manufacture of a pharmaceutical composition for treating a subject suffering from cancer.

18. A method of inhibiting a cyclin-dependent kinase comprising contacting the cyclin-dependent kinase with a compound selected from one of Claims 1-6.

19. A method of Claim 18 wherein said cyclin-dpendent kinase is cdc2.

20. A method of Claim 18 wherein said cyclin-dependent kinase is cdk2.

21. A method of Claim 18 wherein said cyclin-dependent kinase is cdk4 or cdk6.

22. A method of inhibiting a growth factor-mediated tyrosine kinase comprising contacting said growth factor-mediated kinase with a compound selected from one of Claims 1 - 6.

23. A method of Claim 22 wherein said growth factor-mediated tyrosine kinase is platelet derived growth factor (PDGF).

24. A method of Claim 22 wherein said growth factor-mediated tyrosine kinase is fibroblast growth factor (FGF).

## Patentansprüche

1. Verbindung der allgemeinen Formel II 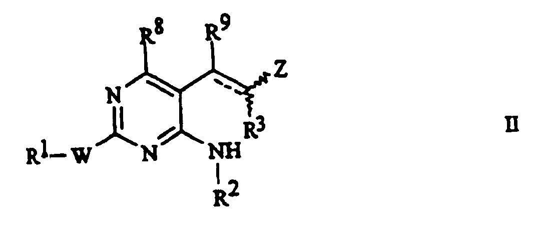 worin
die Punktlinie eine wahlweise Doppelbindung von entweder trans- oder cis-Stereochemie wiedergibt;
W NH, S, SO oder SO₂ darstellt;
Z COOR⁷, CN, CHO, CH₂OR⁷, CH₂NHR⁷, CONHR⁷ oder COR⁷ darstellt und
R¹ und R² unabhängig ausgewählt sind aus der Gruppe, bestehend aus
H,
(CH₂)ₙAr, worin Aryl aus Phenyl oder Naphthyl ausgewählt ist,
(CH₂)ₙHeteroaryl, worin die Heteroarylgruppe 4 bis 9 Ringatome aufweist, wobei 1 bis 4 davon unabhängig aus der Gruppe, bestehend aus O, S und N, ausgewählt sind,
(CH₂)ₙHeterocyclus, der eine wie nachstehend definierte Cycloalkylgruppe mit mindestens einem Heteroatom, ausgewählt aus O, S, NR₂, bedeutet,
C₁-C₁₀-Alkyl,
C₃-C₁₀-Cycloalkyl,
C₂-C₁₀-Alkenyl und
C₂-C₁₀-Alkinyl,
worin n 0, 1, 2 oder 3 ist und die (CH₂)ₙAr, Alkyl-, Cycloalkyl-, Alkenyl- und Alkinylgruppen gegebenenfalls substituiert sind mit Gruppen von
NR⁴R⁵,
N(O)R⁴R⁵,
NR⁴R⁵R⁶Y,
Phenyl,
Phenyl, substituiert mit 1, 2 oder 3 Gruppen, unabhängig ausgewählt aus der Gruppe, bestehend aus C₁-C₁₀-Alkyl, C₁-C₁₀-Alkoxy, Thio, Thio-C₁-C₁₀-alkyl, Hydroxy, -COOR⁷, Amino der Formel -NR⁴R⁵ und T(CH₂)ₘQR⁴ oder T(CH₂)ₘCO₂R⁴, worin m 1 bis 6 ist, T O, S, NR⁴, N(O)R⁴, NR⁴R⁶Y oder CR⁴R⁵ darstellt, Q O, S, NR⁵, N(O)R⁵ oder NR⁵R⁶Y darstellt,
Hydroxy,
C₁-C₁₀-Alkoxy,
Phenoxy,
Thiol,
Thio-C₁-C₁₀-alkyl,
Halogen,
COR⁴,
CO₂R⁴,
CONR⁴R⁵ ,
SO₂NR⁴R⁵,
SO₃R⁴,
PO₃R⁴,
Aldehyd,
Nitril,
Nitro,
Heteroaryloxy, worin Heteroaryl wie vorstehend definiert ist,
T(CH₂)ₘQR⁴, C(O)T(CH₂)ₘQR⁴, NHC(O)T(CH₂)ₘQR⁴ oder T(CH₂)ₘCO₂R⁴, worin m 1 bis 6 ist, T O, S, NR⁴, N(O)R⁴, NR⁴R⁶Y oder CR⁴R⁵ darstellt, Q O, S, NR⁵, N(O)R⁵ oder NR⁵R⁶Y darstellt,
R³ H oder C₁-C₁₀-Alkyl darstellt,
R⁴ und R⁵ unabhängig ausgewählt sind aus der Gruppe, bestehend aus
Wasserstoff,
C₁-C₆-Alkyl
wie vorstehend substituiertem Alkyl
C₂-C₆-Alkenyl,
C₂-C₆-Alkinyl,
wie vorstehend definiertem (CH₂)ₙAr,
C₃-C₁₀-Cycloalkyl oder
R⁴ und R⁵ zusammengenommen mit dem Stickstoff, an den sie gebunden sind, gegebenenfalls einen Ring mit 3 bis 7 Kohlenstoffatomen bilden und der Ring gegebenenfalls 1, 2 oder 3 Heteroatome enthält, ausgewählt aus der Gruppe, bestehend aus
Stickstoff,
substituiertem Stickstoff, wobei "substituierter Stickstoff" Stickstoff bedeutet, der C₁-C₆-Alkyl oder (CH₂)ₙPh trägt, worin n 1, 2 oder 3 ist,
Sauerstoff und
Schwefel;
R⁶ C₁-C₁₀-Alkyl darstellt;
Y ein Halogengegenion darstellt;
R⁷ einen von H, C₁-C₁₀-Alkyl oder Phenyl darstellt;
R⁸ und R⁹ unabhängig
H,
C₁-C₃-Alkyl,
NR⁴R⁵,
N(O)R⁴R⁵,
NR⁴R⁵R⁶Y,
Hydroxy,
wie vorstehend definiertes Alkoxy,
Thiol,
wie vorstehend definiertes Thioalkyl,
Halogen,
COR⁴, CO²R⁴,
CONR⁴R⁵, SO₂NR⁴R⁵, SO₃R⁴, PO₃R⁴, CHO, CN oder NO₂ darstellen
und die pharmazeutisch verträglichen Salze davon.

2. Verbindung nach Anspruch 1, worin W NH darstellt und R⁸ und R⁹ beide Wasserstoff darstellen.

3. Verbindung nach Anspruch 2, worin R¹ Phenyl oder wie vorstehend definiertes substituiertes Phenyl darstellt.

4. Verbindung nach Anspruch 3, nämlich:
3-(4-Ethylamino-2-phenylaminopyrimidin-5-yl) acrylsäureethylester;
3-(4-Amino-2-phenylaminopyrimidin-5-yl) acrylsäureethylester;
3-(4-Ethylamino-2-phenylaminopyrimidin-5-yl) propionsäureethylester;
3-(4-Ethylamino-2-phenylaminopyrimidin-5-yl) acrylnitril und
3-(4-Ethylamino-2-phenylaminopyrimidin-5-yl)-but-2-ensäureethylester.

5. Verbindung nach Anspruch 1, worin W S, SO oder SO₂ darstellt.

6. Verbindung nach Anspruch 5, nämlich:
3-(4-Amino-2-methansulfanyl-pyrimidin-5-yl) acrylsäureethylester;
3-(4-Ethylamino-2-methansulfanyl-pyrimidin-5-yl) acrylsäureethylester;
3-(4-Methylamino-2-methansulfanyl-pyrimidin-5-yl) acrylsäureethylester;
3-(4-Methylamino-2-methansulfanyl-pyrimidin-5-yl)-acrylnitril;
3-(4-Cyclopentylamino-2-methansulfanyl-pyrimidin-5-yl)acrylsäureethylester;
3-(4-Cyclohexylamino-2-methylsulfanyl-pyrimidin-5-yl) - acrylsäureethylester;
3-(4-Cyclopropylamino-2-methylsulfanyl-pyrimidin-5-yl)acrylsäureethylester und
4-[5-(2-Ethoxycarbonyl-vinyl)-2-methylsulfanyl-pyrimidin-4-yl-amino]-piperidin-1-carbonsäureethylester.

7. Pharmazeutische Formulierung, umfassend eine Verbindung, ausgewählt aus Ansprüchen 1 bis 6 in Kombination mit einem pharmazeutisch verträglichen Träger, Verdünnungsmittel oder Exzipienten.

8. Verwendung einer Verbindung, ausgewählt aus einem der Ansprüche 1 bis 6 zur Herstellung einer pharmazeutischen Zusammensetzung zum Bekämpfen von proliferativen Störungen, ausgewählt aus der Gruppe, bestehend aus Krebs, Psoriasis, vaskulärer Glattmuskelproliferation, verbunden mit einer Störung, ausgewählt aus der Gruppe, bestehend aus Arteriosklerose, postchirurgischer vaskulärer Stenose und Restenose, bei Säugern.

9. Verwendung einer Verbindung, ausgewählt aus einem der Ansprüche 1 bis 6 zur Herstellung einer pharmazeutischen Zusammensetzung zum Hemmen einer Cyclin-abhängigen Kinase.

10. Verwendung nach Anspruch 9, wobei die Cyclin-abhängige Kinase cdc2 ist.

11. Verwendung nach Anspruch 9, wobei die Cyclin-abhängige Kinase cdk2 ist.

12. Verwendung nach Anspruch 9, wobei die Cyclin-abhängige Kinase cdk4 oder cdk6 ist.

13. Verwendung einer Verbindung, ausgewählt aus einem der Ansprüche 1 bis 6 zur Herstellung einer pharmazeutischen Zusammensetzung zum Hemmen einer Wachstumsfaktor-vermittelten Tyrosinkinase.

14. Verwendung nach Anspruch 13, wobei die Wachstumsfaktorvermittelte Tyrosinkinase Platelet-derived growth-Faktor (PDGF) ist.

15. Verwendung nach Anspruch 13, wobei die Wachstumsfaktorvermittelte Tyrosinkinase Fibroblastenwachstumsfaktor (FGF) ist.

16. Verwendung einer Verbindung, ausgewählt aus einem der Ansprüche 1 bis 6 zur Herstellung einer pharmazeutischen Zusammensetzung zur Behandlung eines Patienten, der unter Krankheiten, verursacht durch vaskuläre Glattmuskelzellproliferation, leidet.

17. Verwendung einer Verbindung, ausgewählt aus einem der Ansprüche 1 bis 6 zur Herstellung einer pharmazeutischen Zusammensetzung zum Behandeln eines Patienten, der unter Krebs leidet.

18. Verfahren zum Hemmen einer Cyclin-abhängigen Kinase, umfassend In-Kontakt-Bringen der Cyclin-abhängigen Kinase mit einer Verbindung, ausgewählt aus einem der Ansprüche 1 bis 6.

19. Verfahren nach Anspruch 18, wobei die Cyclin-abhängige Kinase cdc2 ist.

20. Verfahren nach Anspruch 18, wobei die Cyclin-abhängige Kinase cdk2 ist.

21. Verfahren nach Anspruch 18, wobei die Cyclin-abhängige Kinase cdk4 oder cdk6 ist.

22. Verfahren zum Hemmen einer Wachstumsfaktor-vermittelten Tyrosinkinase, umfassend In-Kontakt-Bringen der Wachstumsfaktor-vermittelten Kinase mit einer Verbindung, ausgewählt aus einem der Ansprüche 1 bis 6.

23. Verfahren nach Anspruch 22, wobei die Wachstumsfaktorvermittelte Tyrosinkinase Platelet-derived growth-Faktor (PDGF) ist.

24. Verfahren nach Anspruch 22, wobei die Wachstumsfaktorvermittelte Tyrosinkinase Fibroblastenwachstumsfaktor (FGF) ist.

## Revendications

1. Composé ayant la formule générale II : 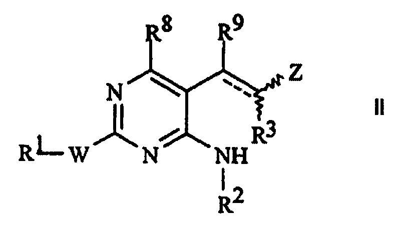 dans laquelle :
la ligne en pointillés représente une double-liaison facultative de stéréochimie trans ou cis ;
W représente NH, S, SO ou SO₂ ;
Z représente COOR⁷, CN, CHO, CH₂OR⁷, CH₂NHR⁷, CONHR⁷ ou COR⁷ ;
R¹ et R² sont indépendamment sélectionnés dans le groupe consistant en :
H,
(CH₂)ₙAr, où aryle est sélectionné parmi phényle ou naphtyle
(CH₂)ₙhétéroaryle, où le groupe hétéroaryle a de 4 à 9 atomes de noyau, parmi lesquels de 1 à 4 sont indépendamment sélectionnés dans le groupe consistant en O, S et N, (CH₂)ₙhétérocycle, ce dernier terme désignant un groupe cycloalkyle tel que défini ci-dessous ayant au moins un hétéroatome sélectionné parmi O, S, NR²,
alkyle en C₁-C₁₀,
cycloalkyle en C₃-C₁₀,
alkényle en C₂-C₁₀ et
alkynyle en C₂-C₁₀,
n représentant 0, 1, 2 ou 3 et les groupes (CH₂)ₙAr, alkyle, cycloalkyle, alkényle et alkynyle étant facultativement substitués par les groupes suivants :
NR⁴R⁵,
N(O)R⁴R⁵,
NR⁴R⁵R⁶Y,
phényle,
phényle substitué par 1, 2 ou 3 groupes indépendamment sélectionnés dans le groupe consistant en alkyle en C₁-C₁₀, alkoxy en C₁-C₁₀, thio, thio(alkyle en C₁-C₁₀), hydroxy, -COOR⁷, amino de formule -NR⁴R⁵ et T(CH₂)ₘR⁴ ou T(CH₂)ₘCO₂R⁴, où m représente un chiffre entre 1 et 6, T représente O, S, NR⁴, N(O)R⁴, NR⁴R⁶Y ou CR⁴R⁵, Q représente O, S, NR⁵, N(O)R⁵ ou NR⁵R⁶Y,
hydroxy,
alkoxy en C₁-C₁₀,
phénoxy,
thiol,
thio(alkyle en C₁-C₁₀),
halogéno,
COR⁴
CO₂R⁴,
CONR⁴R⁵,
SO₂NR⁴R⁵,
SO₃R⁴,
PO₃R⁴,
aldéhyde
nitrile,
nitro,
hétéroaryloxy, hétéroaryle étant tel que défini ci-dessus,
T(CH₂)ₘQR⁴, C(O)T(CH₂)ₘQR⁴, NHC(O)T(CH₂)ₘQR⁴ ou T(CH₂)ₘCO₂R⁴, où m représente un chiffre entre 1 et 6, T représente O, S, NR⁴, N(O)R⁴, NR⁴R⁶Y ou CR⁴R⁵, Q représente O, S, NR⁵, N(O)R⁵ ou NR⁵R⁶Y,
R³ représente H ou alkyle en C₁-C₁₀,
R⁴ et R⁵ étant indépendamment sélectionnés dans le groupe consistant en :
l'hydrogène,
alkyle en C₁-C₆,
alkyle substitué comme défini ci-dessus,
alkényle en C₂-C₆,
alkynyle en C₂-C₆,
(CH₂)ₙAr tel que défini ci-dessus,
cycloalkyle en C₃-C₁₀,
ou R⁴ et R⁵, pris ensemble avec l'atome d'azote auquel ils sont liés forment facultativement un noyau ayant de 3 à 7 atomes de carbone, ledit noyau contenant facultativement 1, 2 ou 3 hétéroatomes sélectionnés dans le groupe consistant en :
l'azote,
un atome d'azote substitué, "azote substitué" signifiant que l'atome d'azote porte un groupe alkyle en C₁-C₆ ou (CH₂)ₙPh, n représentant 1, 2 ou3,
l'oxygène et
le soufre
R⁶ représentant alkyle en C₁-C₁₀ ;
Y étant un contre-ion halogéné ;
R⁷ représentant l'un de H, alkyle en C₁-C₁₀ ou phényle ;
R⁸ et R⁹ représentent indépendamment :
H,
alkyle en C₁-C₃,
NR⁴R⁵,
N(O)R⁴R⁵,
NR⁴R⁵R⁶Y,
hydroxy,
alcoxy tel que défini ci-dessus,
thiol,
thioalkyle tel que défini ci-dessus,
halogéno,
COR⁴, CO₂R⁴,
CONR⁴R⁵, SO₂NR⁴R⁵, SO₃R⁴, PO₃R⁴, CHO, CN ou NO₂ ;
et sels pharmaceutiquement acceptables correspondants.

2. Composé selon la revendication 1, dans lequel W représente NH et R⁸ et R⁹ représentent tous deux l'hydrogène.

3. Composé selon la revendication 2, dans lequel R¹ est un groupe phényle ou phényle substitué tel que défini ci-dessus.

4. Composé selon la revendication 3 qui est :
le 3-(4-éthylamino-2-phénylamino-pyrimidin-5-yl)acrylate d'éthyle ;
le 3-(4-amino-2-phénylamino-pyrimidin-5-yl)acrylate d'éthyle ;
l'ester éthylique de l'acide 3-(4-éthylamino-2-phénylamino-pyrimidin-5-yl)propionique ;
le 3-(4-éthylamino-2-phénylamino-pyrimidin-5-yl)acrylonitrile ; et
l'ester éthylique de l'acide 3-(4-éthylamino-2-phénylamino-pyrimidin-5-yl)but-2-énoïque.

5. Composé selon la revendication 1, dans lequel W représente S, SO ou SO₂.

6. Composé selon la revendication 5 qui est :
le 3-(4-amino-2-méthanesulfanyl-pyrimidin-5-yl)acrylate d'éthyle ;
le 3-(4-éthylamino-2-méthanesulfanyl-pyrimidin-5-yl)acrylate d'éthyle ;
le 3-(4-méthylamino-2-méthanesulfanyl-pyrimidin-5-yl)acrylate d'éthyle ;
le 3-(4-méthylamino-2-méthanesulfanyl-pyrimidin-5-yl)acrylonitrile ;
le 3-(4-cyclopentylamino-2-méthanesulfanyl-pyrimidin-5-yl)acrylate d'éthyle ;
l'ester éthylique de l'acide 3-(4-cyclohexylamino-2-méthylsulfanyl-pyrimidin-5-yl)acrylique ;
l'ester éthylique de l'acide 3-(4-cyclopropylamino-2-méthylsulfanyl-pyridimin-5-yl)acrylique ; et
l'ester éthylique de l'acide 4-[5-(2-éthoxycarbonyl-vinyl)-2-méthylsulfanyl-pyrimidin-4-ylamino]pipéridine-1-carboxylique.

7. Formulation pharmaceutique comprenant un composé sélectionné parmi les revendications 1-6 en combinaison avec un support, diluant ou excipient pharmaceutiquement acceptable.

8. Utilisation d'un composé sélectionné parmi les revendications 1-6 pour la fabrication d'une composition pharmaceutique pour la maîtrise de troubles prolifératifs sélectionnés dans le groupe consistant en le cancer, le psoriasis, la prolifération des cellules de muscles lisses vasculaires associée à un trouble sélectionné dans le groupe consistant en l'athérosclérose, la sténose vasculaire post-chirurgicale et la resténose chez les mammifères.

9. Utilisation d'un composé sélectionné parmi les revendications 1-6 pour la fabrication d'une composition pharmaceutique pour inhiber une kinase cycline-dépendante.

10. Utilisation selon la revendication 9, dans laquelle ladite kinase cycline-dépendante est la cdc2.

11. Utilisation selon la revendication 9, dans laquelle ladite kinase cycline-dépendante est la cdk2.

12. Utilisation selon la revendication 9, dans laquelle ladite kinase cycline-dépendante est la cdk4 ou la cdk6.

13. Utilisation d'un composé sélectionné parmi les revendications 1-6 pour la fabrication d'une composition pharmaceutique pour inhiber une tyrosine kinase médiée par un facteur de croissance.

14. Utilisation selon la revendication 13, dans laquelle ladite tyrosine kinase médiée par un facteur de croissance est le facteur de croissance dérivé des plaquettes (PDGF).

15. Utilisation selon la revendication 13, dans laquelle ladite tyrosine kinase médiée par un facteur de croissance est le facteur de croissance des fibroblastes (FGF).

16. Utilisation d'un composé sélectionné parmi les revendications 1-6 pour la fabrication d'une composition pharmaceutique pour traiter un sujet souffrant de maladies provoquées par une prolifération des cellules de muscles lisses vasculaires.

17. Utilisation d'un composé sélectionné parmi les revendications 1-6 pour la fabrication d'une composition pharmaceutique pour traiter un sujet souffrant du cancer.

18. Procédé d'inhibition d'une kinase cycline-dépendante comprenant la mise en contact de la kinase cycline-dépendante avec un composé sélectionné parmi l'une des revendications 1-6.

19. Procédé selon la revendication 18, dans lequel ladite kinase cycline-dépendante est la cdc2.

20. Procédé selon la revendication 18, dans lequel ladite kinase cycline-dépendante est la cdk2.

21. Procédé selon la revendication 18, dans lequel ladite kinase cycline-dépendante est la cdk4 ou la cdk6.

22. Procédé d'inhibition d'une tyrosine kinase médiée par un facteur de croissance comprenant la mise en contact de ladite kinase médiée par un facteur de croissance avec un composé sélectionné parmi l'une des revendications 1-6.

23. Procédé selon la revendication 22, dans lequel ladite tyrosine kinase médiée par un facteur de croissance est le facteur de croissance dérivé des plaquettes (PDGF).

24. Procédé selon la revendication 22, dans lequel ladite tyrosine kinase médiée par un facteur de croissance est le facteur de croissance des fibroblastes (FGF).
